# EUROPEAN PATENT APPLICATION

(11) **EP 4 400 148 A1**
(43) Date of publication of application: **17.07.2024**
(21) Application number: 22867129.3
(22) Date of filing: 08.08.2022
(51) Int. Cl.: A61M 25/02, A61M 39/06

(54) **OPENING/CLOSING MECHANISM FOR HEMOSTASIS VALVE, MEDICAL CONNECTOR AND FIXING MECHANISM FOR ELONGATED MEDICAL INSTRUMENT**

(30) Priority: 09.09.2021 JP 2021146544
(71) Applicant: Asahi Intecc Co., Ltd., Seto-shi, Aichi 489-0071 (JP)
(72) Inventor: OSHIMIZU Ryuya, Seto-shi, Aichi 489-0071 (JP); MATSUO Kenichi, Seto-shi, Aichi 489-0071 (JP); HONGO Yutaka, Seto-shi, Aichi 489-0071 (JP); CHIKANO Ichiro, Seto-shi, Aichi 489-0071 (JP)
(74) Representative: Winter, Brandl - Partnerschaft mbB
(86) International application number: PCT/JP2022/030209
(87) International publication number: WO 2023/037807

(57) **Abstract**

The operability of an opening/closing mechanism is improved. A through-member is positioned along a first direction in a first position that closes a hemostasis valve, and a second position that presses and opens the hemostasis valve. The operating member displaces the through-member toward a distal side by sliding along a second direction not parallel to the first direction. A biasing member biases the through-member toward a proximal side. A motion restricting groove on the through-member includes a closed position portion fitted with a pin when the through-member is in the first position; a first top portion fitted with the pin when the through-member subsequently reaches the distal side of the second position; an open holding portion fitted with the pin when the through-member is in the second position; and a second top portion fitted with the pin when the through-member subsequently reaches the distal side of the second position.

## Description

### TECHNICAL FIELD

The technique disclosed herein relates to an opening/closing mechanism for a hemostasis valve, a fixing mechanism for a long medical device, and a medical connector.

### BACKGROUND ART

A Y connector is a medical connector that is used by being connected to a guiding catheter. A Y connector has a main pipe portion and a branched pipe portion that is branched from the main pipe portion, where a long medical device such as a guide wire or a catheter is introduced into the guiding catheter via the main pipe portion, and a liquid agent such as a contrast medium or physiological saline is provided via the branched pipe portion. AY connector is provided with an opening/closing mechanism for opening and closing a hemostasis valve that suppresses the outflow of blood via the lumen of the main pipe portion, and a fixing mechanism for fixing a long medical device.

An opening/closing mechanism for a hemostasis valve of a conventional Y connector is provided with a through-member (opener) formed having a through-hole that is coaxial with the lumen of the main pipe portion, and as a result of performing an operation of pressing the through-member in a direction parallel to the axial direction of the lumen of the main pipe portion, it is possible to switch between an open state in which the through-member presses the hemostasis valve and opens the hemostasis valve, and a closed state in which the through-member separates from the hemostasis valve and closes the hemostasis valve (see Patent Literature 1). Furthermore, the fixing mechanism for a long medical device in the Y connector disclosed in Patent Literature 1 is provided with an elastic fixing valve formed having a through-hole that the long medical device is inserted through, and as a result of performing a rotation operation with respect to a screw to move the screw in the axial direction, which accordingly moves a pusher in the axial direction, it is possible to switch between a fixed state, in which the fixing valve undergoes elastic deformation as a result of being pressed by the pusher, which causes a reduction in the inner diameter of the through-hole of the fixing valve and causes the long medical device to be fixed, and an unfixed state in which the fixing valve is not pressed by the pusher, which results in an expansion in the inner diameter of the through-hole of the fixing valve and the release of the fixing of the long medical device.

### CITATION LIST

### Patent Literature

Patent Literature 1: Japanese Patent No. 5249049

### SUMMARY OF INVENTION

### TECHNICAL PROBLEM

In the configuration of the Y connector described above, because the opening and closing operation of the hemostasis valve is an operation of pressing the through-member in the axial direction of the lumen of the main pipe portion, the operation can sometimes be difficult to perform. Furthermore, because the fixing/unfixing operation of the long medical device is an operation that causes the screw to rotate, the operation is complicated, and it is not possible to grasp, visually or by feel, the degree to which the long medical device is fixed.

A technique capable of solving the problems described above is disclosed herein.

### SOLUTION TO PROBLEM

The technique disclosed herein can be achieved, for example, as the following aspects.

(1) An opening/closing mechanism for a hemostasis valve disclosed herein comprises a housing, a hemostasis valve, a through-member, an operating member, and a biasing member. The housing is a tubular member formed having a lumen that communicates with a distal end side opening and a proximal end side opening. The hemostasis valve is attached inside the housing and is normally in a closed state, and is switched to an open state when pressed from a proximal end side such that a through-hole is formed that communicates with the distal end side opening of the housing. The through-member is accommodated inside the housing so as to be capable of sliding along a first direction, being an extending direction of the lumen further toward a proximal end side than the hemostasis valve. The through-member is formed having a through-hole that communicates with the proximal end side opening of the housing. The through-member is capable of being positioned in a first position that places the hemostasis valve in the closed state, and a second position, being a position further toward a distal end side along the first direction than the first position, which places the hemostasis valve in the open state by pressing the hemostasis valve, and communicates the through-hole of the hemostasis valve and the through-hole of the through-member. The surface of the through-member is formed having a motion restricting groove that is connected over an entire circumference. The operating member is accommodated inside the housing so as to be capable of sliding in a second direction that is not parallel to the first direction, in a state where one part is exposed from the housing. The operating member is configured so as to be capable of pressing and displacing the through-member toward a distal end side by sliding along the second direction toward an inner side of the housing. The biasing member biases the through-member toward a proximal end side. This opening/closing mechanism for a hemostasis valve is further provided with a pin that is attached to the housing such that an end portion is loosely fitted to the motion restricting groove. The motion restricting groove is formed having a closed position portion, a first top portion, an open holding portion, and a second top portion. The closed position portion is a part to which the end portion of the pin is fitted without restricting a movement of the through-member toward a distal end side when the through-member is in a first state at the first position. The first top portion is a part to which the end portion of the pin is fitted without restricting a movement of the through-member toward a proximal end side when the through-member is in a second state, in which the through-member has been pressed from the first state by the operating member and reaches a position further toward a distal end side than the second position. The open holding portion is a part to which the end portion of the pin is fitted such that a movement of the through-member toward a proximal end side is restricted when the through-member is in a third state, in which the through-member has been displaced from the second state by a biasing force of the biasing member to the second position. The second top portion is a part to which the end portion of the pin is fitted without restricting a movement of the through-member toward a proximal end side when the through-member is in a fourth state, in which the through-member has been pressed from the third state by the operating member reaches a position further toward a distal end side than the second position.
   As described above, in this opening/closing mechanism for a hemostasis valve, when the through-member is positioned in the first position and the hemostasis valve is in the closed state, and the operating member receives a pressing force and slides along a second direction toward the inner side of the housing, the through-member is pressed by the operating member and moves to a position further on the distal end side than the second position, and as a result, the end portion of the pin reaches the first top portion, which is a part of the motion restricting groove to which the end portion of the pin is fitted without restricting the movement of the through-member toward the proximal end side. Then, when the pressing force with respect to the operating member is released, the through-member moves toward the proximal end side to the second position due to the biasing force of the biasing member, and the hemostasis valve switches from the closed state to the open state. At this time, as a result of the end portion of the pin moving from the first top portion of the motion restricting groove to the open holding portion, because the movement of the through-member toward the proximal end side is restricted and the through-member is maintained in the second position, the open state of the hemostasis valve is maintained. Furthermore, when the operating member once again receives a pressing force and slides along the second direction toward the inner side of the housing, the through-member is pressed by the operating member and moves to a position further toward the distal end side than the second position, and as a result, the end portion of the pin reaches the second top portion, which is a part of the motion restricting groove to which the end portion of the pin is fitted without restricting the movement of the through-member toward the proximal end side. Then, when the pressing force with respect to the operating member is released, the through-member moves toward the proximal end side to the first position due to the biasing force of the biasing member, and the hemostasis valve switches from the open state to the closed state. Here, in this opening/closing mechanism for a hemostasis valve, because the operating member is capable of sliding along the second direction that is not parallel to the first direction, being the extending direction of the lumen of the housing, the opening and closing operation of the hemostasis valve becomes an operation of pressing the operating member in the second direction, which is not parallel to the extending direction of the lumen of the housing. As a result, according to this opening/closing mechanism for a hemostasis valve, it is possible for an operator to easily perform the opening and closing operation of the hemostasis valve with the thumb while gripping a device to which this opening/closing mechanism is provided, and the operability of the opening/closing mechanism can be improved.
(2) The opening/closing mechanism for a hemostasis valve described above may have a configuration in which the biasing member has a cylindrical shape, and a surface of the through-member facing the biasing member is formed having a protrusion that is inserted into a hollow portion of the biasing member. According to this opening/closing mechanism for a hemostasis valve, it is possible to easily and accurately perform the positioning between the through-member and the biasing member, and it is also possible to effectively transmit the biasing force of the biasing member to the through-member, which enables the accuracy of the motion of the opening/closing mechanism to be improved.
(3) In the opening/closing mechanism for a hemostasis valve above, an angle formed between the first direction and the second direction may be 35 degrees or more and 55 degrees or less. According to this opening/closing mechanism for a hemostasis valve, it is possible for an operator to very easily perform the opening and closing operation of the hemostasis valve with the thumb while gripping a device to which this opening/closing mechanism is provided, and the operability of the opening/closing mechanism can be effectively improved.
(4) The opening/closing mechanism for a hemostasis valve described above may have a configuration in which the biasing member and the operating member are disposed so as to face each other in the first direction. According to this opening/closing mechanism for a hemostasis valve, it is possible to efficiently transmit the pressing force applied to the operating member to the biasing member and deform the biasing member, and the operability of the opening/closing mechanism can be efficiently improved.
(5) In the opening/closing mechanism for a hemostasis valve above, the through-member may include a main body portion that is formed having a through-hole, and a flange portion that protrudes from the main body portion in a third direction, which is orthogonal to the first direction, and the operating member may make contact with a surface of the flange portion on a proximal end side, while also being configured so as to be capable of a relative sliding movement with respect to the through-member along the third direction. According to this opening/closing mechanism for a hemostasis valve, the sliding of the operating member along the second direction that is not parallel to the first direction that is the extending direction of the lumen of the housing and the sliding of the through-member in the extending direction can be efficiently converted, and the operability of the opening/closing mechanism can be effectively improved.
(6) In the opening/closing mechanism for a hemostasis valve above, an angle formed between the first direction and the second direction may be substantially 90 degrees. According to this opening/closing mechanism for a hemostasis valve, it is possible for an operator to very easily perform the opening and closing operation of the hemostasis valve with the thumb and/or index finger while gripping a device to which this opening/closing mechanism is provided, and the operability of the opening/closing mechanism can be effectively improved.
(7) The opening/closing mechanism for a hemostasis valve described above may have a configuration in which the operating member is formed of a first piece and a second piece that face each other in the second direction, and the first piece and the second piece sliding along the second direction so as to approach each other, which causes the through-member to be pressed and displaced toward a distal end side. According to this opening/closing mechanism for a hemostasis valve, it is possible for an operator to very easily and stably perform the opening and closing operation of the hemostasis valve by performing an operation of pinching the first piece and the second piece between the thumb and index finger while gripping a device to which this opening/closing mechanism is provided, and the operability of the opening/closing mechanism can be effectively improved.
(8) The opening/closing mechanism for a hemostasis valve described above have a configuration in which the first piece and the second piece each have an operating member side contact surface, being a surface that is parallel to a fourth direction which is orthogonal to both the first direction and the second direction, that is not parallel to both the first direction and the second direction, and the through-member has a through-member side first contact surface, being a surface that is parallel to the fourth direction and not parallel to both the first direction and the second direction, that makes contact with the operating member side contact surface of the first piece, and a through-member side second contact surface, being a surface that is parallel to the fourth direction and not parallel to both the first direction and the second direction, that makes contact with the operating member side contact surface of the second piece. According to this opening/closing mechanism for a hemostasis valve, the sliding of the operating member along the second direction, which is substantially orthogonal to the extending direction of the lumen of the housing, and the sliding of the through-member in the extending direction can be efficiently converted, and the operability of the opening/closing mechanism can be effectively improved.
(9) The fixing mechanism for a long medical device disclosed herein includes a housing, a cylindrical body, a pressing member, an operating member, a force transmission member, and a holding mechanism. The housing is a tubular member formed having a lumen that communicates with a distal end side opening and a proximal end side opening. The cylindrical body is attached inside the housing, and is a flexible member formed having a through-hole into which the long medical device is inserted. The through-hole of the cylindrical body communicates with the distal end side opening and the proximal end side opening of the housing. The pressing member is accommodated inside the housing and is capable of sliding along a sixth direction, which is a direction orthogonal to a fifth direction that is parallel to an axis of the cylindrical body. The pressing member is capable of being positioned in a fifth position, and a sixth position that is displaced from the fifth position along the sixth direction, at which a part of the cylindrical body excluding both end portions is deformed by being pressed from an outer peripheral side. The operating member is a member that is capable of sliding along a seventh direction that is not parallel to the fifth direction. The force transmission member is disposed so as to be capable of sliding in the seventh direction between the pressing member and the operating member, and is a member that transmits, to the pressing member, a force that moves the operating member along the seventh direction so as to approach the pressing member. The force transmission member is capable of being positioned in a seventh position that places the pressing member in the fifth position, and an eighth position that presses the pressing member and positions the pressing member in the sixth position. The holding mechanism 260 switches states between a state in which the force transmission member is held in the seventh position and a state in which the force transmission member is held in the eighth position each time the operating member slides along the seventh direction. This fixing mechanism for a long medical device is configured such that, when the pressing member is positioned in the sixth position, an inner peripheral surface of the deformed cylindrical body is pressed against the long medical device, which causes the long medical device to become fixed by the cylindrical body.
   In this way, in this fixing mechanism for a long medical device, the holding mechanism switches between a state in which the force transmission member is held in the seventh position and a state in which the force transmission member is held in the eighth position each time the operating member slides along the seventh direction. When the holding mechanism switches with the sliding of the operating member to a state in which the force transmission member is held in the eighth position, the pressing member moves from the fifth position to the sixth position, and the cylindrical body undergoes elastic deformation and the long medical device is fixed by the cylindrical body. At this time, because the holding mechanism holds the force transmission member in the eighth position, the fixed state of the long medical device is maintained. Furthermore, when the holding mechanism switches with the sliding of the operating member again to a state in which the force transmission member is held in the seventh position, the pressing member moves from the sixth position to the fifth position, and shape of the cylindrical body is restored which releases the fixing of the long medical device. At this time, because the holding mechanism holds the force transmission member in the seventh position, a state in which the fixing of the long medical device has been released is maintained. Moreover, in this fixing mechanism for a long medical device, because the operating member is capable of sliding along the seventh direction, which is not parallel to a direction parallel to an axis of the cylindrical body, the fixing operation and unfixing operation of the long medical device becomes an operation of pressing the operating member in the seventh direction, which is not parallel to a direction parallel to an axis of the cylindrical body. Therefore, according to this fixing mechanism for a long medical device, it is possible to achieve the fixing and unfixing of the long medical device with a simple operation in which the operator presses the operating member with the thumb while gripping a device provided with this fixing mechanism, and because the degree to which the long medical device is fixed can be grasped visually or by feel, the operability of the fixing mechanism can be improved.
(10) In the fixing mechanism for a long medical device above, the holding mechanism may include a cylindrical biasing member that biases the force transmission member in a direction approaching the pressing member, and a surface of the force transmission member facing the biasing member may be formed having a protrusion that is inserted into a hollow portion of the biasing member. According to this fixing mechanism for a long medical device, it is possible to easily and accurately perform the positioning between the force transmission member and the biasing member, and it is also possible to effectively transmit the biasing force of the biasing member to the force transmission member, which enables the accuracy of the motion of the fixing mechanism to be improved.
(11) In the fixing mechanism for a long medical device above, an angle formed between the fifth direction and the seventh direction may be 35 degrees or more and 55 degrees or less. According to this fixing mechanism for a long medical device, it is possible to very easily operate the operating member with the thumb while gripping a device to which this fixing mechanism is provided, and the operability of the fixing mechanism can be effectively improved.
(12) In the fixing mechanism for a long medical device above, the force transmission member may make contact with a surface of the pressing member, while also being configured to be capable of a relative sliding movement with respect to the pressing member along the fifth direction. According to this fixing mechanism for a long medical device, the sliding of the force transmission member along the seventh direction and the sliding of the pressing member along the seventh direction can be efficiently converted, and the operability of the fixing mechanism can be effectively improved.
(13) Another fixing mechanism for a long medical device disclosed herein includes a housing, a cylindrical body, a distal end side connection portion, a proximal end side connection portion, and a pressing member. The housing is a tubular member formed having a lumen that communicates with a distal end side opening and a proximal end side opening, and which is capable of accommodating the long medical device in the lumen. The flexible cylindrical body is attached inside the housing and is formed having a first through-hole into which the long medical device is inserted, the first through-hole communicating with the distal end side opening and the proximal end side opening of the housing. The distal end side connection portion connects a distal end portion of the cylindrical body to the housing, and is formed having a second through-hole that communicates with the first through-hole of the cylindrical body. The proximal end side connection portion connects a proximal end portion of the cylindrical body to the housing, and is formed having a third through-hole that communicates with the first through-hole of the cylindrical body. The pressing member presses a pressed part of the cylindrical body excluding both end portions from a direction that is not parallel to an axis of the cylindrical body, and is accommodated inside the housing. An area of the second through-hole of the distal end side connection portion in a transverse cross-section of the fixing mechanism and an area of the third through-hole in the transverse cross-section of the proximal end side connection portion are larger than an area of the first through-hole of the pressed part in the transverse cross-section.

In this way, in this fixing mechanism for a long medical device, when the pressing member presses the pressed part of the cylindrical body excluding both end portions, the cylindrical body undergoes elastic deformation and the long medical device is fixed by the cylindrical body. At this time, because the area of the second through-hole of the distal end side connection portion and the area of the third through-hole of the proximal end side connection portion are larger than the area of the first through-hole of the pressed part of the cylindrical body, even when the long medical device is fixed as a result of the pressing member pressing the pressed part of the cylindrical body, the long medical device is prevented from being pressed against the inner peripheral surface of the distal end side connection portion and the proximal end side connection portion, and damage to the long medical device can be more effectively suppressed.

The technique disclosed in herein can be realized in various forms, and can be realized in a form such as an opening/closing mechanism for a hemostasis valve, a fixing mechanism for a long medical device, a medical connector provided with an opening/closing mechanism for a hemostasis valve and/or a fixing mechanism for a long medical device, or a medical device provided with a medical connector.

### BRIEF DESCRIPTION OF DRAWINGS

FIG. 1 is an explanatory diagram showing a configuration of a medical connector according to a first embodiment.
FIG. 2 is an explanatory diagram showing a configuration of the medical connector shown in FIG. 1.
FIG. 3 is an explanatory diagram showing a configuration of an opening/closing mechanism for a hemostasis valve of a medical connector.
FIG. 4 is an explanatory diagram showing a configuration of the opening/closing mechanism shown in FIG. 3.
FIG. 5 is an explanatory diagram showing a configuration of an opening/closing mechanism.
FIG. 6 is a cross-sectional perspective view showing a configuration of a housing of an opening/closing mechanism.
FIG. 7 is a perspective view showing an external configuration of a through-member and an operating member of an opening/closing mechanism.
FIG. 8 is an explanatory diagram showing the position of a pin in a motion restricting groove of an opening/closing mechanism.
FIG. 9 is an explanatory diagram showing the position of a pin in a motion restricting groove.
FIG. 10 is an explanatory diagram showing a configuration of a fixing mechanism according to the first embodiment.
FIG. 11 is an explanatory diagram showing a configuration of the fixing mechanism shown in FIG. 1.
FIG. 12 is a perspective view showing an external configuration of a cylindrical body, a pressing member, and a force transmission member of a fixing mechanism.
FIG. 13 is a perspective view showing an external configuration of an operating member and a holding mechanism of a fixing mechanism.
FIG. 14 is a perspective view showing an external configuration of an outer cylinder of a fixing mechanism.
FIG. 15 is an explanatory diagram showing an external configuration of a medical connector according to a second embodiment.
FIG. 16 is an explanatory diagram showing a configuration of an opening/closing mechanism for a hemostasis valve of the medical connector shown in FIG. 15.
FIG. 17 is an explanatory diagram showing a configuration of the opening/closing mechanism shown in FIG. 16.
FIG. 18 is an explanatory diagram showing a configuration of an opening/closing mechanism.
FIG. 19 is a perspective view showing an external configuration of a through-member and an operating member of an opening/closing mechanism.
FIG. 20 is a perspective view showing an external configuration of a through-member and an operating member.
FIG. 21 is an explanatory diagram showing the position of a pin in a motion restricting groove of an opening/closing mechanism.
FIG. 22 is an explanatory diagram showing the position of a pin in a motion restricting groove.
FIG. 23 is an external perspective view showing a configuration of an operating member of an opening/closing mechanism.
FIG. 24 is an explanatory diagram showing a configuration of a longitudinal cross-section of a medical connector according to a third embodiment.
FIG. 25 is an explanatory diagram showing a state of the fixing mechanism shown in

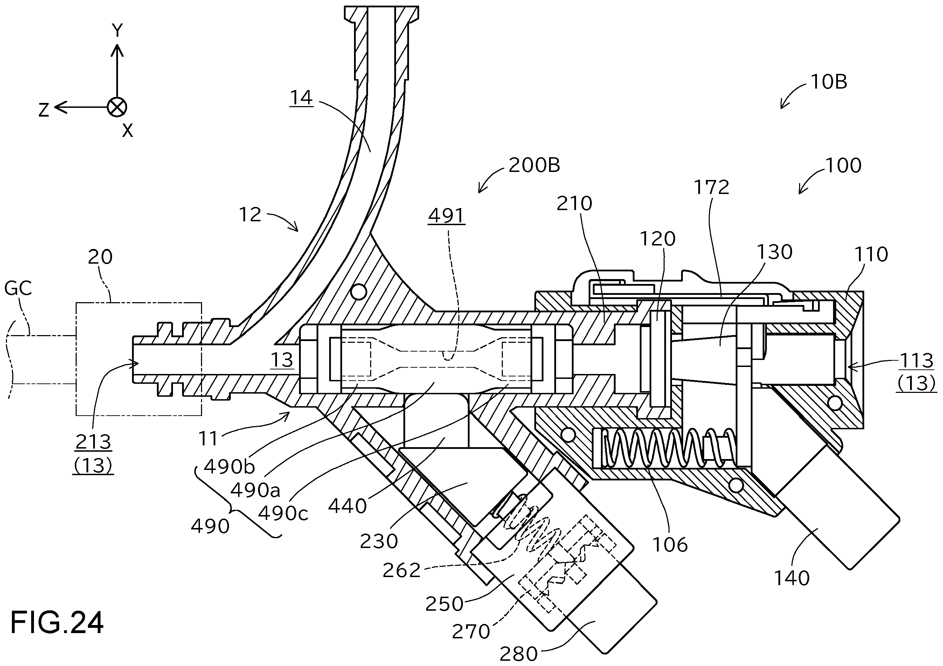

FIG. 24 where fixing of a long medical device has been released.

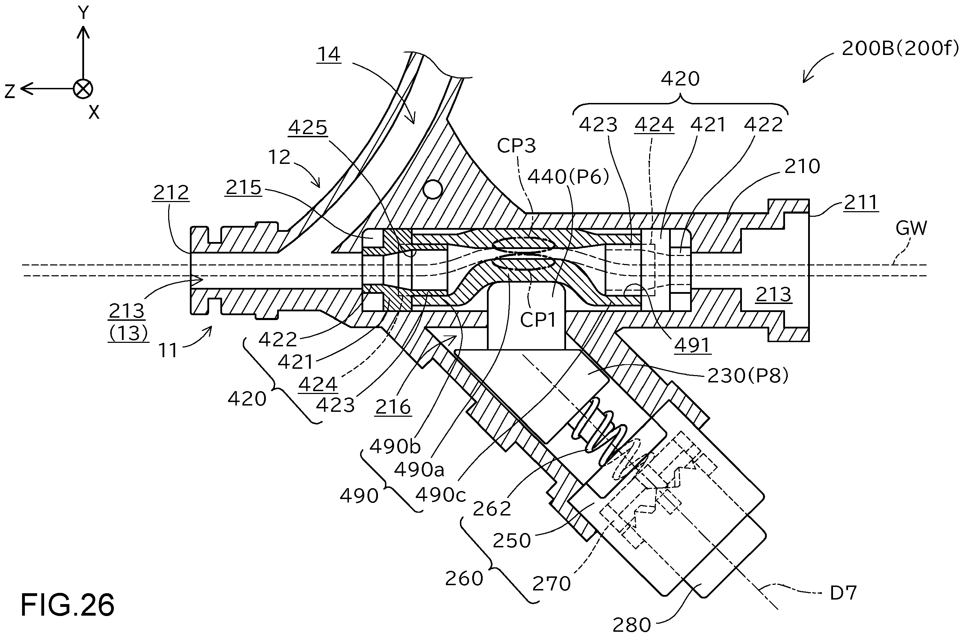

FIG. 26 is an explanatory diagram showing a state of the fixing mechanism shown in
FIG. 24 where a long medical device has been fixed.

### EMBODIMENTS OF THE INVENTION

### A. First Embodiment:

### A-1. Configuration of Medical Connector:

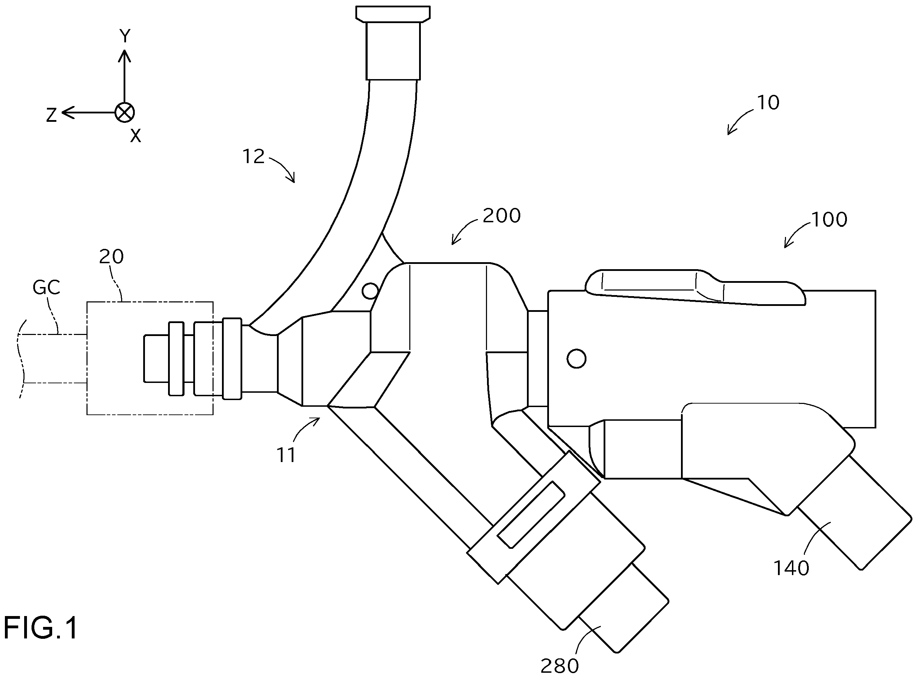

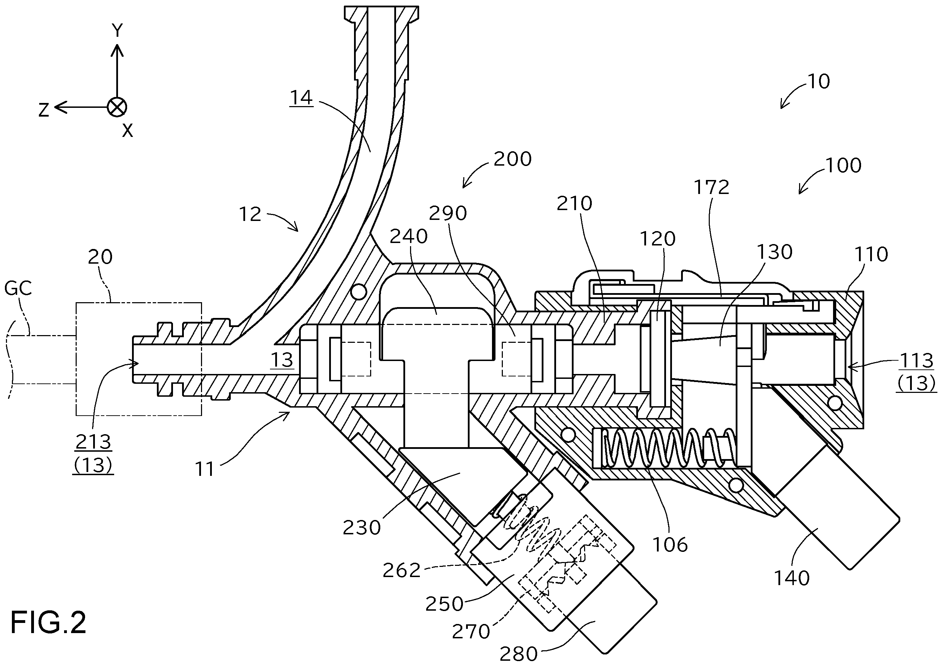

FIGS. 1 and 2 are explanatory diagrams showing a configuration of a medical connector in a first embodiment. FIG. 1 schematically shows an external configuration of a medical connector 10, and FIG. 2 shows a configuration of a longitudinal cross-section (YZ cross-section) of the medical connector 10. The medical connector 10 is a Y connector that is used by being connected to a guiding catheter GC via a rotator 20. Herein, the side of the medical connector 10 to which the guiding catheter GC is connected (positive Z-axis direction side) is referred to as the distal end side, and the opposite side (negative Z-axis direction side) is referred to as the proximal end side. Furthermore, in the medical connector 10 and each of the constituent members, the end on the distal end side is referred to as the "distal end", the distal end and the vicinity thereof are referred to as the "distal end portion", the end on the proximal end side is referred to as the "proximal end", and the proximal end and the vicinity thereof are referred to as the "proximal end portion". Moreover, for convenience of description, the Z-axis direction is also referred to as the front-rear direction, the Y-axis direction is also referred to as the up-down direction, the positive Y-axis direction is also referred to as the upward direction, the negative Y-axis direction is also referred to as the downward direction, and the X-direction is also referred to as the left-right direction. However, the posture of the medical connector 10 is not limited to this. Further, in the following description, the side surfaces of some members may be shown in the cross-sectional views such as shown in FIG. 2.

The medical connector 10 includes a tubular main pipe portion 11 extending in the front-rear direction, and a tubular branched pipe portion 12 branching from the vicinity of a distal end portion of the main pipe portion 11 and diagonally extends toward an upper proximal end side. The main pipe portion 11 is formed having a lumen 13 that extends in the front-rear direction and passes through the main pipe portion 11, and a long medical device (not illustrated) such as a guide wire or a catheter is introduced into the guiding catheter GC via the lumen 13. Furthermore, the branched pipe portion 12 is formed having a lumen 14 that communicates with the lumen 13 of the main pipe portion 11, and a liquid agent such as a contrast medium or physiological saline is provided via the lumen 14 from a liquid agent supply apparatus (not illustrated) connected to an end portion of the branched pipe portion 12.

The medical connector 10 includes an opening/closing mechanism 100 for a hemostasis valve, and a fixing mechanism 200 for a long medical device. The opening/closing mechanism 100 is provided further toward the proximal end side than the fixing mechanism 200. The opening/closing mechanism 100 constitutes a portion of the proximal end side of the main pipe portion 11 of the medical connector 10, and the fixing mechanism 200 constitutes a portion of the distal end side of the main pipe portion 11 of the medical connector 10 and the branched pipe portion 12. Hereinafter, the configurations of the opening/closing mechanism 100 and the fixing mechanism 200 will be described in turn.

The medical connector 10 is used by being gripped by an operator such as a physician. For example, the operator supports the main pipe portion 11 of the medical connector 10 in the posture shown in FIG. 1 such that the four fingers from the index finger to the little finger of the hand that is not grasping the long medical device cover the main pipe portion 11 from above, and grasps the medical connector 10 such that the thumb is positioned in the vicinity of an operating member 140 of the opening/closing mechanism 100 or an operating member 280 of the fixing mechanism 200.

### A-2. Configuration of Opening/Closing Mechanism:

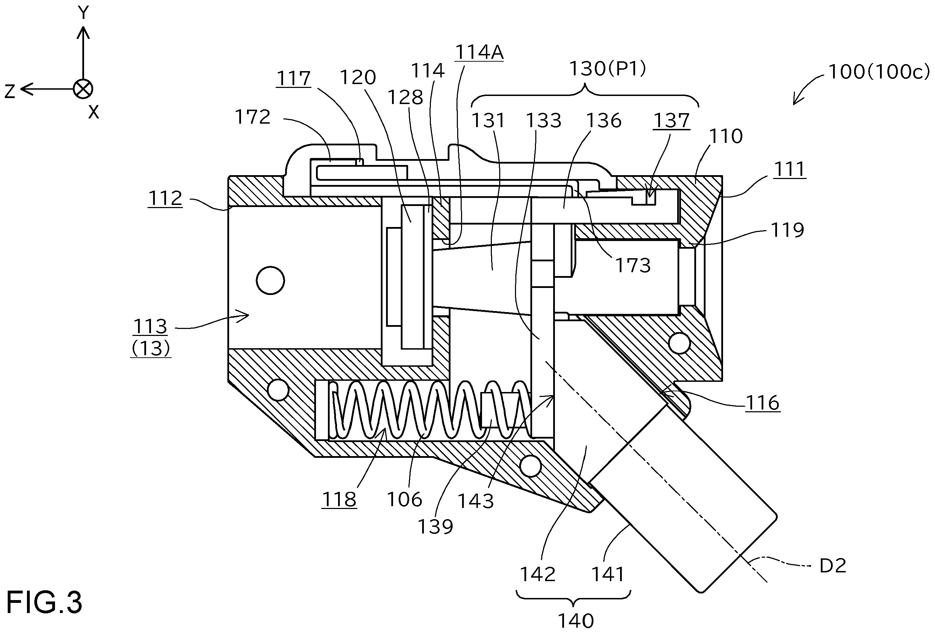

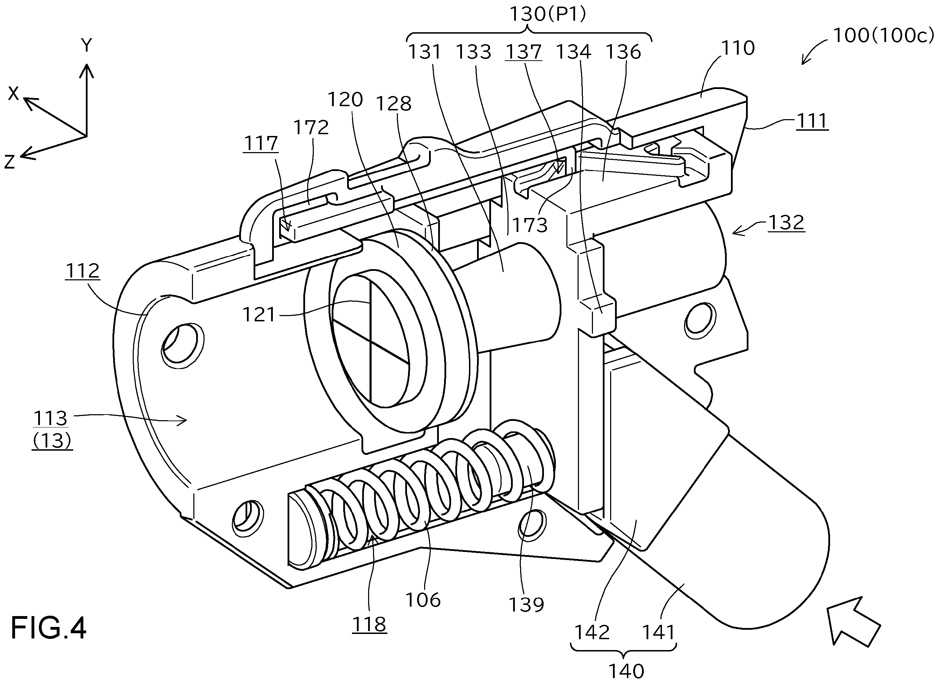

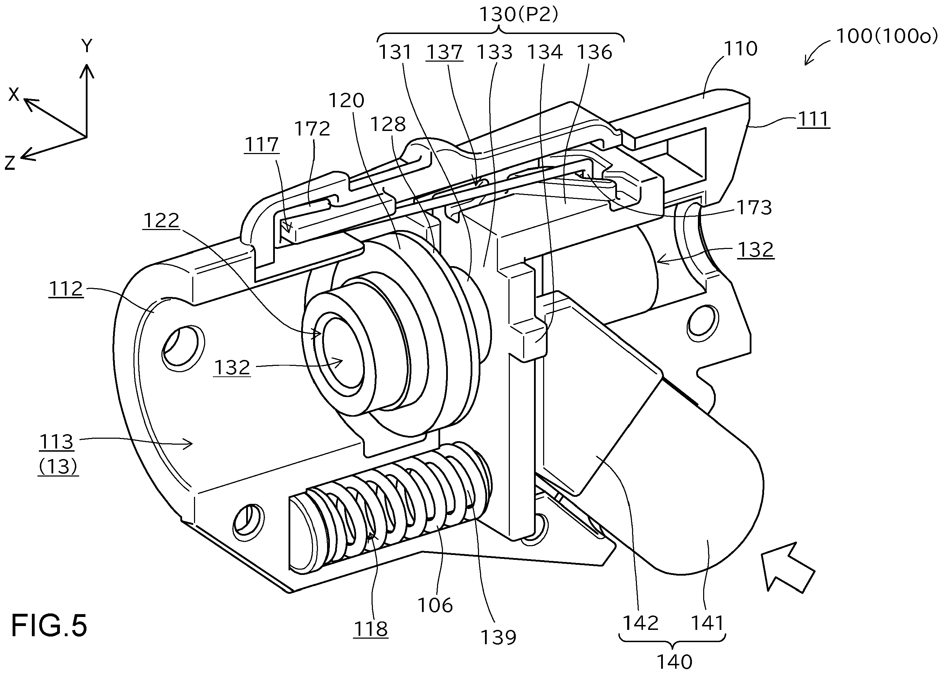

Next, the configuration of the opening/closing mechanism 100 for a hemostasis valve 120 will be described. FIGS. 3 to 5 are explanatory diagrams showing the configuration of the opening/closing mechanism 100 in the first embodiment. FIG. 3 shows the configuration in a longitudinal cross-section (YZ cross-section) of the opening/closing mechanism 100, and FIGS. 4 and 5 show a cross-sectional perspective view thereof. FIGS. 3 and 4 show the opening/closing mechanism 100 in a state in which the hemostasis valve 120 is closed (hereinafter referred to as "closed state opening/closing mechanism 100c"), and FIG. 5 shows the opening/closing mechanism 100 in a state in which the hemostasis valve 120 is open (hereinafter referred to as "open state opening/closing mechanism 100o).

The opening/closing mechanism 100 is a mechanism for opening and closing the hemostasis valve 120, which suppresses the outflow of blood via the lumen 13 of the main pipe portion 11 of the medical connector 10. The opening/closing mechanism 100 is a mechanism that switches between a state in which the hemostasis valve 120 is closed or a state in which the hemostasis valve 120 is open state each time the operator performs a pressing operation with respect to the operating member 140. The opening/closing mechanism 100 includes a housing 110, a hemostasis valve 120, a through-member 130, an operating member 140, a biasing member 106, and a pin 172.

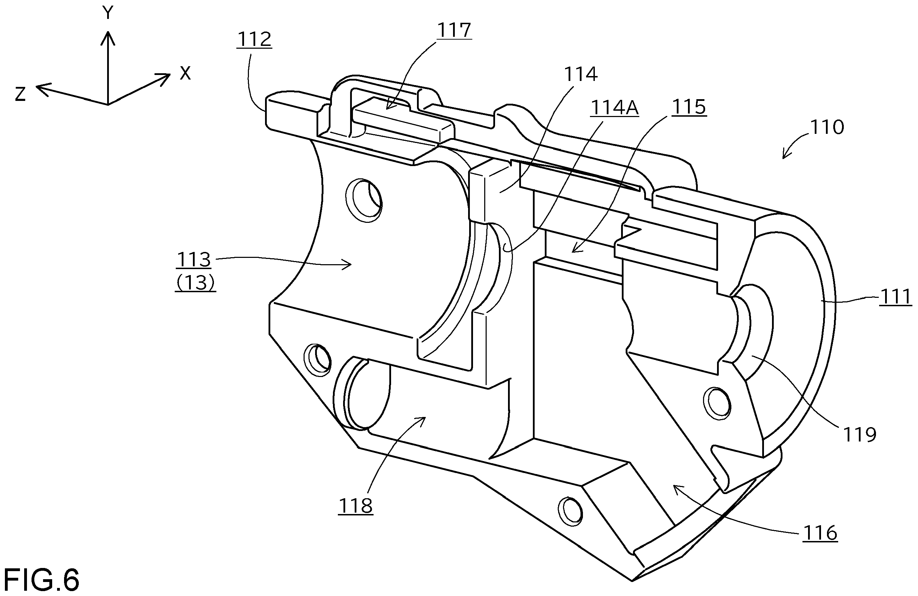

FIG. 6 is a cross-sectional perspective view showing a configuration of the housing 110. The housing 110 is formed having a distal end side opening 112 and a proximal end side opening 111, and is a tubular member formed having a lumen 113 that communicates with the distal end side opening 112 and the proximal end side opening 111. The lumen 113 is a through-hole extending in the front-rear direction (Z-axis direction), and constitutes a portion of the lumen 13 of the main pipe portion 11 of the medical connector 10. The housing 110, for example, is formed of a resin. In addition, as shown in FIG. 2, the proximal end portion of the fixing mechanism 200 is inserted and fixed inside the lumen 113. The Z-axis direction is an example of a first direction in the claims.

A substantially flat plate-shaped partition wall 114 that is substantially orthogonal to the front-rear direction is formed in the vicinity of the center of the inside of the housing 110 in the front-rear direction. The partition wall 114 is formed having a through-hole 114A that passes through the front-rear direction and constitutes a portion of the lumen 113. The transverse cross-sectional shape of the through-hole 114A, for example, is substantially circular. Furthermore, a side surface inside the housing 110 is formed having guide grooves 115 that extend from the position of the partition wall 114 toward the proximal end side. The guide grooves 115 are formed as a pair that face each other in the left-right direction (X-axis direction). Moreover, the proximal end portion inside the housing 110 is formed having an operating member accommodating space 116 that communicates with the lumen 113, and extends in a diagonal direction toward the lower proximal end side and is open at the surface of the housing 110. In addition, a biasing member accommodating space 118 extending in the front-rear direction is formed in a lower part of the inside of the housing 110. Also, a groove 117 for accommodating the pin 172 is formed in an upper part of the inside of the housing 110.

As shown in FIGS. 3 to 5, the hemostasis valve 120 is a substantially circular plate-shaped member, and is formed of an elastic material such as silicone rubber. The hemostasis valve 120 is fixed at a position further toward the distal end side than the partition wall 114 inside the housing 110. A slit 121 is formed at a position substantially at the center of the hemostasis valve 120 when viewed in the Z-axis direction (FIG. 4). The hemostasis valve 120 is normally in the closed state in which the slit 121 is closed and the valve is closed (FIGS. 3 and 4). When the hemostasis valve 120 is in the closed state, the lumen 113 of the housing 110 (i.e., the lumen 13 of the main pipe portion 11) is closed by the hemostasis valve 120, and the outflow of blood to the proximal end side of the hemostasis valve 120 via the lumen 113 is suppressed. Furthermore, when the hemostasis valve 120 is pressed from the proximal end side, each piece divided by the slit 121 elastically deforms and is displaced toward the distal end side, and the hemostasis valve 120 switches to the open state, in which a through-hole 122 is formed that passes through the hemostasis valve 120 in front-rear direction (FIG. 5). In the open state, the through-hole 122 communicates with the distal end side opening 112 via the lumen 113. Therefore, when the hemostasis valve 120 is in the open state, the lumen 113 is not closed at the position of the hemostasis valve 120, and is in a pass-through state. When the pressing force from the proximal end side is no longer applied, the hemostasis valve 120 is elastically deformed and returns to the closed state. Also, in the present embodiment, a substantially annular boss 128 is disposed between the hemostasis valve 120 and the partition wall 114.

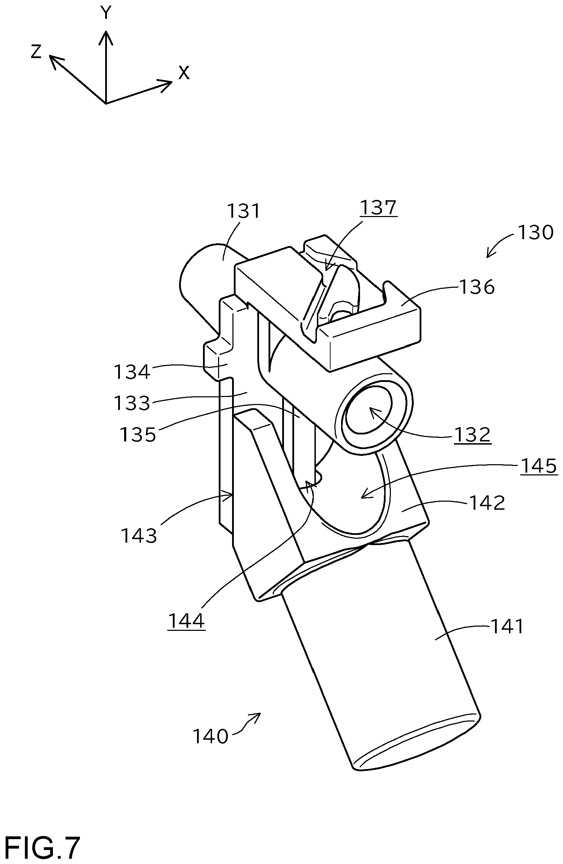

FIG. 7 is a perspective view showing an external configuration of the through-member 130 and the operating member 140. As shown in FIGS. 3 to 5, and FIG. 7, the through-member 130 is a member formed having a through-hole 132 extending in the front-rear direction, and for example, is formed of a resin. More specifically, the through-member 130 includes a substantially cylindrical main body portion 131 formed having the through-hole 132 extending in the front-rear direction, a substantially flat plate-shaped flange portion 133 that downwardly protrudes from the vicinity of the center of the main body portion 131 in the front-rear direction, and is substantially orthogonal to the front-rear direction, and a substantially flat plate-shaped upper wall portion 136 that is disposed above the main body portion 131, and is substantially orthogonal to the up-down direction. The flange portion 133 also protrudes above the main body portion 131, and the upper wall portion 136 has a shape that extends from the upper end of the flange portion 133 toward the proximal end side. As shown in FIG. 7, a pair of guide protrusions 134 that protrude in the left-right direction are formed on a side surface of the flange portion 133, and a continuous guide protrusion 135 extending in the up-down direction is formed on the proximal end side surface of the flange portion 133. The upper surface of the upper wall portion 136 is formed having a motion restricting groove 137. The motion restricting groove 137 is a so-called heart cam groove, and is a groove having a heart shape that is connected over the entire circumference. The up-down direction is an example of a third direction in the claims.

The through-member 130 is accommodated inside the housing 110 further toward the proximal end side than the hemostasis valve 120. In the through-member 130, the through-hole 132 of the main body portion 131 communicates with the proximal end side opening 111 of the housing 110. Furthermore, the through-hole 132 and the lumen 113 are coaxial with each other.

In the through-member 130, the pair of guide protrusions 134 formed on the flange portion 133 (FIG. 7) are fitted into the pair of guide grooves 115 formed in the housing 110 (FIG. 6). As a result, the through-member 130 is capable of sliding in the front-rear direction with the guide grooves 115 as a guide in a state in which the position with respect to the housing 110 in the up-down direction and the left-right direction is fixed. The through-member 130 is positioned such that the main body portion 131 faces the through-hole 114A of the partition wall 114 in the front-rear direction. The through-member 130, which is capable of sliding in the front-rear direction, has the distal end portion of the main body portion 131 inserted into the through-hole 114A, and can be positioned in a pressing position P2 that presses the hemostasis valve 120 and places the hemostasis valve 120 in the open state (the state shown in FIG. 5), and a non-pressing position P1 that does not press the hemostasis valve 120 and places the hemostasis valve 120 in the closed state (the state shown in FIGS. 3 and 4). As shown in FIG. 5, in the state in which the through-member 130 is positioned in the pressing position P2, the through-hole 122 formed in the hemostasis valve 120 and the through-hole 132 of the through-member 130 communicate with each other. The distal end side of the through-member 130 is capable of moving to a position in which the flange portion 133 makes contact with the partition wall 114, and the proximal end side is capable of moving to a position in which the proximal end of the through-member 130 makes contact with a protrusion 119 formed on the proximal end portion of the housing 110 (FIGS. 3 and 6). The pressing position P2 is an example of a second position in the claims, and the non-pressing position P1 is an example of a first position in the claims.

The biasing member 106 is a member that biases the through-member 130 toward the proximal end side, and is accommodated in the biasing member accommodating space 118 of the housing 110. In the present embodiment, a substantially hollow cylindrical spring is used as the biasing member 106. The spring is formed of a metal such as stainless steel. When the through-member 130, which is biased toward the proximal end side by the biasing member 106, is not receiving a force from the operating member 140 and the pin 172, it is positioned in the non-pressing position P1 (FIGS. 3 and 4) that does not press the hemostasis valve 120. Note that, in the present embodiment, a projection-shaped protrusion 139 is formed on a surface of the flange portion 133 of the through-member 130 facing the biasing member 106, and the protrusion 139 is inserted into a hollow portion of the biasing member 106.

The pin 172 is a long member having a narrow diameter, and is formed of a metal such as stainless steel. The pin 172 is attached to the housing 110 with a posture that extends in the front-rear direction. The end portion of the pin 172 on the distal end side is accommodated in the groove 117 formed in the housing 110, and the position is fixed. The end portion 173 of the pin 172 on the proximal end side is bent and loosely fitted into the motion restricting groove 137 that is formed in the upper wall portion 136 of the through-member 130. The position of the end portion 173 in the motion restricting groove 137 changes with the sliding of the through-member 130 in the front-rear direction (Z-axis direction).

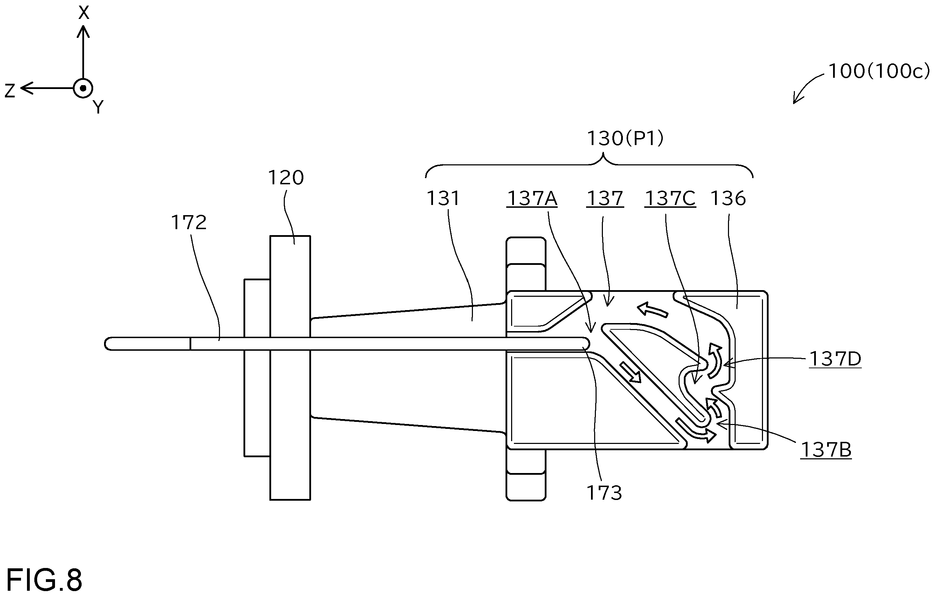

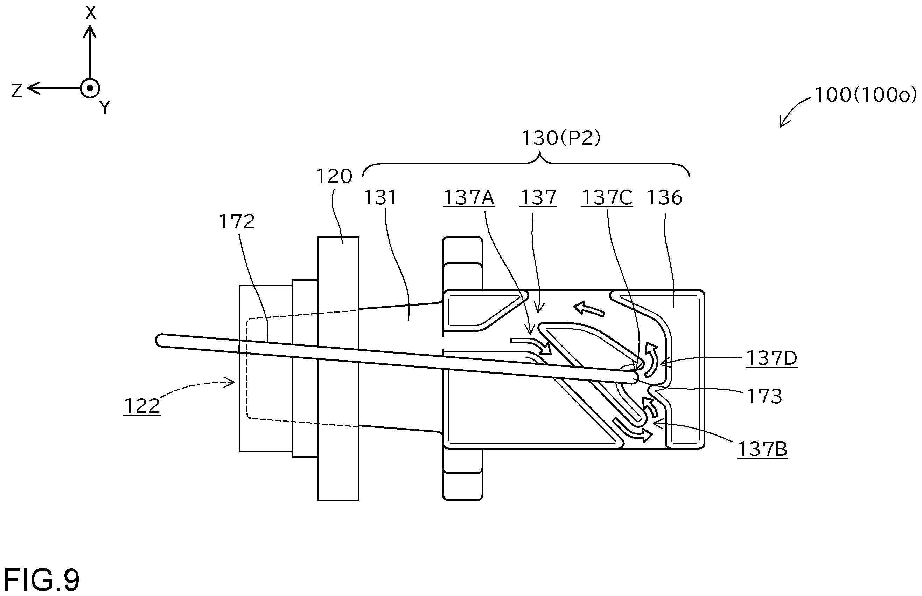

FIGS. 8 and 9 are explanatory diagrams showing the position of the pin 172 in the motion restricting groove 137. FIGS. 8 and 9 show an upper surface configuration of the pin 172, the through-member 130, and the hemostasis valve 120. FIG. 8 shows the closed state opening/closing mechanism 100c in which the through-member 130 is in the non-pressing position P1 and the hemostasis valve 120 is closed, and FIG. 9 shows the open state opening/closing mechanism 100o in which the through-member 130 is in the pressing position P2 and the through-hole 122 is formed in the hemostasis valve 120.

As indicated by the white arrows in FIGS. 8 and 9, as the through-member 130 slides in the front-rear direction (Z-axis direction), the end portion 173 of the pin 172 swings in the left-right direction (X-axis direction), and relatively moves in one direction (counterclockwise in the illustrated example) in the motion restricting groove 137. That is to say, as shown in FIG. 8, in the first state in which the through-member 130 is in the non-pressing position P1, the end portion 173 is positioned at a bottom portion (hereinafter, referred to as "closed position portion 137A") of the heart shape of the motion restricting groove 137. In this state, the movement of the through-member 130 in the front-rear direction is not restricted by the pin 172. When the through-member 130 moves toward the distal end side (positive Z-axis direction) from the first state, the end portion 173 also relatively moves in the motion restricting groove 137 toward the proximal end side. In the second state, in which the through-member 130 is displaced from the non-pressing position P1 to the pressing position P2, and has reached a position further toward the distal end side than the pressing position P2, the end portion 173 is positioned at one of a pair of top portions (hereinafter, referred to as "first top portion 137B") that sandwich the heart-shaped valley portion of the motion restricting groove 137. In this state, the movement of the through-member 130 toward the proximal end side is not restricted by the pin 172. In the third state, in which the through-member 130 has moved to the pressing position P2 by slightly returning toward the proximal end side from the second state, as shown in FIG. 9, the end portion 173 relatively moves toward the distal end side in the motion restricting groove 137, and reaches the heart-shaped valley portion (hereinafter, referred to as "open holding portion 137C") of the motion restricting groove 137. In this state, the movement of the through-member 130 toward the proximal end side is restricted due to the interference between the pin 172 and the motion restricting groove 137, and a state in which the through-member 130 is positioned in the pressing position P2 is maintained. In the fourth state, in which the through-member 130 has reached a position further toward the distal end side than the pressing position P2 by moving slightly toward the distal end side from the third state, the end portion 173 relatively moves toward the proximal end side in the motion restricting groove 137, and is positioned at the other of the pair of top portions (hereinafter, referred to as "second top portion 137D") that sandwich the heart-shaped valley portion of the motion restricting groove 137. In this state, the movement of the through-member 130 toward the proximal end side is not restricted by the pin 172. When the through-member 130 moves toward the proximal end side to the non-pressing position P1 from the fourth state, the end portion 173 also relatively moves toward the distal end side in the motion restricting groove 137 and returns to the closed position portion 137A of the motion restricting groove 137 (FIG. 8).

As shown in FIGS. 3 to 5 and FIG. 7, the operating member 140 is a long member extending in a direction (hereinafter referred to as "operation direction D2") that is not parallel to the front-rear direction (Z-axis direction). In the present embodiment, the operation direction D2 is a direction at an angle of 35 degrees or more and 55 degrees or less with respect to the front-rear direction, and for example, is a direction at an angle of 45 degrees with respect to the front-rear direction (a direction inclined at 45 degrees from the upper distal end side toward the lower proximal end side). The operating member 140 is accommodated in the operating member accommodating space 116 of the housing 110 such that it is capable of sliding in the operation direction D2. In the present embodiment, the operating member 140 is disposed so as to face the biasing member 106 in the front-rear direction. The operating member 140, for example, is formed of a resin. The operation direction D2 is an example of a second direction in the claims.

The operating member 140 includes a substantially cylindrical operation accepting portion 141, and a substantially polyhedral connection portion 142 that is positioned on the distal end side thereof. A part of the operation accepting portion 141 on the proximal end side is exposed from the housing 110, which enables a pressing operation to be made by an operator such as a physician. A distal end surface 143 of the connection portion 142 has a planar shape that is substantially orthogonal to the front-rear direction, and makes contact with the proximal end side surface of the flange portion 133 of the through-member 130. As shown in FIG. 7, the distal end surface 143 is formed having a guide groove 144 extending in the up-down direction, and the guide groove 144 is fitted with a guide protrusion 135 that is formed on the proximal end side surface of the flange portion 133. Furthermore, an upper surface of the connection portion 142 is formed having a recess 145 that is capable of accommodating the proximal end portion of the main body portion 131 of the through-member 130.

As a result of the operating member 140 having the configuration described above, the operating member 140 makes contact with the proximal end side surface of the flange portion 133 of the through-member 130, while also being capable of a relative sliding movement with respect to the through-member 130 along the up-down direction. That is, as shown in FIGS. 3 and 4, when the through-member 130 is in the non-pressing position P1 that does not press the hemostasis valve 120, the operating member 140 is in a retracted position where it is retracted to the lower proximal end side. In addition, when the operating member 140 slides from the retracted position along the operation direction D2 toward the inner side of the housing 110 (that is, diagonally upward), the operating member 140 maintains a state in which the guide protrusion 135 of the through-member 130 is fitted to the guide groove 144 of the operating member 140, while also undergoing a relative upward sliding movement with respect to the through-member 130, which presses and displaces the through-member 130 toward the distal end side. The sliding range of the operating member 140 is set to a range that enables the through-member 130 to be moved further toward the distal end side than the pressing position P2.

### A-3. Motion of Opening/Closing Mechanism:

Next, the motion of the opening/closing mechanism 100 for the hemostasis valve 120 will be described. In the initial state, as shown in FIGS. 3 and 4, the through-member 130 is positioned in the non-pressing position P1, in which the through-member 130 receives a biasing force from the biasing member 106 and does not press the hemostasis valve 120, and the operating member 140 indirectly receives the biasing force via the through-member 130 and is positioned in the retracted position. In this state, the hemostasis valve 120 is closed, and the opening/closing mechanism 100 is the closed state opening/closing mechanism 100c. At this time, as shown in FIG. 8, the end portion 173 of the pin 172 is positioned in the closed position portion 137A of the motion restricting groove 137 in the upper wall portion 136 of the through-member 130.

For example, when the thumb of the operator that is gripping the medical connector 10 applies a valve-opening operation of pressing the operation accepting portion 141 of the operating member 140 along the operation direction D2 toward the inner side of the housing 110 (that is, diagonally upward), the operating member 140 slides diagonally upward along the operation direction D2. Accordingly, the through-member 130 is pressed by the operating member 140 and moves toward the distal end side. Furthermore, the end portion 173 of the pin 172 relatively moves toward the proximal end side in the motion restricting groove 137 formed in the through-member 130. When the through-member 130 moves a fixed distance or more toward the distal end side, the distal end portion of the main body portion 131 of the through-member 130 presses the hemostasis valve 120 and places the hemostasis valve 120 in the open state. When the valve-opening operation is released by the operator after the through-member 130 reaches a position further toward the distal end side than the pressing position P2 and the end portion 173 reaches the first top portion 137B of the motion restricting groove 137, as shown in FIG. 9, the through-member 130 returns slightly toward the proximal end side due to the biasing force of the biasing member 106, and the end portion 173 also relatively moves toward the distal end side in the motion restricting groove 137 and reaches the open holding portion 137C. In this state, because the movement of the through-member 130 toward the proximal end side is restricted due to the interference between the pin 172 and the motion restricting groove 137, a state in which the through-member 130 is positioned in the pressing position P2 is maintained. As a result, the opening/closing mechanism 100 becomes the open state opening/closing mechanism 100o shown in FIG. 5.

Furthermore, when the opening/closing mechanism 100 is the open state opening/closing mechanism 100o, for example, when the thumb of the operator applies, in the same manner as the valve-opening operation, a valve-closing operation of pressing the operation accepting portion 141 of the operating member 140 along the operation direction D2 toward the inner side of the housing 110 (that is, diagonally upward), the operating member 140 slides diagonally upward along the operation direction D2. Accordingly, the through-member 130 is pressed by the operating member 140 and moves to a position further toward the distal end side than the pressing position P2. Furthermore, the end portion 173 relatively moves toward the proximal end side and reaches the second top portion 137D from the open holding portion 137C in the motion restricting groove 137. In this state, the movement of the through-member 130 toward the proximal end side is not restricted by the pin 172. Consequently, when the valve-closing operation is released by the operator, the through-member 130 moves to the proximal end side due to the biasing force of the biasing member 106, and returns to the non-pressing position P1. Accordingly, as shown in FIG. 8, the end portion 173 relatively moves toward the distal end side in the motion restricting groove 137 and returns to the closed position portion 137A. As a result, the hemostasis valve 120 is placed in the closed state, and the opening/closing mechanism 100 becomes the closed state opening/closing mechanism 100c shown in FIGS. 3 and 4.

In this way, the state of the opening/closing mechanism 100 switches states between the closed state opening/closing mechanism 100c and the open state opening/closing mechanism 100o each time the pressing operation (the valve-opening operation and the valve-closing operation) is performed with respect to the operating member 140.

### A-4. Technical Effects of Opening/Closing Mechanism:

As described above, in the opening/closing mechanism 100 of the present embodiment, the operating member 140 is pressed when the through-member 130 is positioned in the non-pressing position P1, and the hemostasis valve 120 is switched from the open to the closed state, or from the closed to the open state. At this time, because the operating member 140 is capable of sliding along the operation direction D2, which is not parallel to the extending direction (Z-axis direction) of the lumen 113 of the housing 110, the opening and closing operation of the hemostasis valve 120 becomes an operation of pressing the operating member 140 in the operation direction D2, which is not parallel to the extending direction of the lumen 113 of the housing 110. As a result, according to the opening/closing mechanism 100 of the present embodiment, it is possible for an operator to easily perform the opening and closing operation of the hemostasis valve 120 with the thumb while gripping the medical connector 10, and the operability of the opening/closing mechanism 100 can be improved.

The biasing member 106 is cylindrical, and the protrusion 139 that is inserted into the hollow portion of the biasing member 106 is formed on the surface of the through-member 130 facing the biasing member 106. Therefore, according to the opening/closing mechanism 100, it is possible to easily and accurately perform the positioning between the through-member 130 and the biasing member 106, and it is also possible to effectively transmit the biasing force of the biasing member 106 to the through-member 130, which enables the accuracy of the motion of the opening/closing mechanism 100 to be improved.

The angle formed between the extending direction (Z-axis direction) of the lumen 113 of the housing 110 and the operation direction D2 of the operating member 140 is 35 degrees or more and 55 degrees or less. As a result, according to the opening/closing mechanism 100 of the present embodiment, it is possible for an operator to very easily perform the opening and closing operation of the hemostasis valve 120 with the thumb while gripping the medical connector 10, and the operability of the opening/closing mechanism 100 can be effectively improved.

The biasing member 106 and the operating member 140 are disposed so as to face each other in the extending direction (Z-axis direction) of the lumen 113 of the housing 110. As a result, according to the opening/closing mechanism 100 of the present embodiment, it is possible to efficiently transmit the pressing force applied to the operating member 140 to the biasing member 106 and deform the biasing member 106, and the operability of the opening/closing mechanism 100 can be efficiently improved.

The through-member 130 includes the main body portion 131 in which the through-hole 132 is formed, and the flange portion 133 that protrudes in the up-down direction from the main body portion 131, and the operating member 140 makes contact with the surface on the proximal end side of the flange portion 133 of the through-member 130 and is configured to be capable of a relative sliding movement with respect to the through-member 130 along the up-down direction. Therefore, according to the opening/closing mechanism 100 of the present embodiment, the sliding of the operating member 140 along the operation direction D2 not parallel to the extending direction (Z-axis direction) of the lumen 113 of the housing 110 and the sliding of the through-member 130 in the extending direction (Z-axis direction) can be efficiently converted, and the operability of the opening/closing mechanism 100 can be effectively improved.

### A-5. Configuration of Fixing Mechanism:

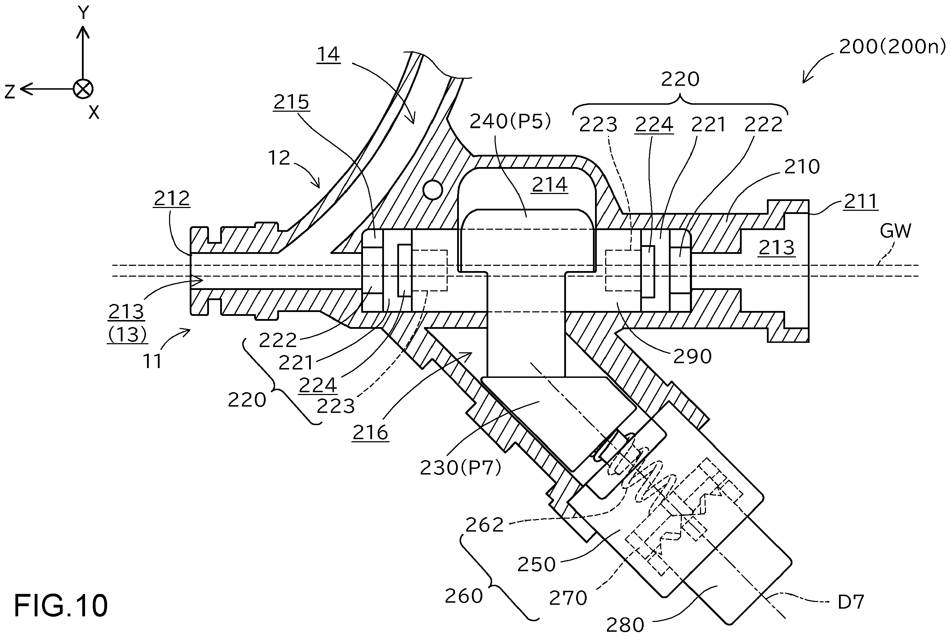

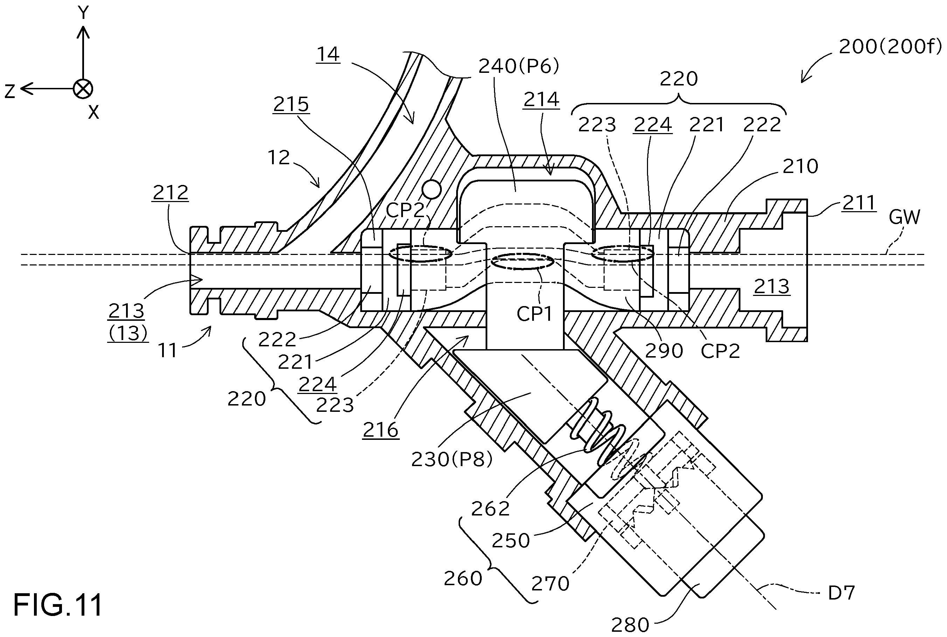

Next, the configuration of the fixing mechanism 200 for a long medical device will be described. FIGS. 10 and 11 are explanatory views of the configuration, and show a configuration (for some members, a configuration of the side surface) of the longitudinal cross-section (YZ cross-section) of the fixing mechanism 200. FIG. 10 shows the fixing mechanism 200 in a state in which the fixing of a long medical device such as the guide wire GW has been released (hereinafter referred to as "released state fixing mechanism 200n"), and FIG. 11 shows the fixing mechanism 200 in a state in which a long medical device has been fixed (hereinafter referred to as "fixed state fixing mechanism 200f').

The fixing mechanism 200 is a mechanism for fixing, and releasing the fixing of a long medical device inserted through the lumen 13 of the main pipe portion 11 (FIG. 2) of the medical connector 10. The fixing mechanism 200 is a mechanism that switches states between a fixed state in which the long medical device is fixed and an unfixed state in which the fixing of the long medical device is released each time a pressing operation is performed by an operator with respect to the operating member 280. The fixing mechanism 200 includes a housing 210, a cylindrical body 290, a pressing member 240, an operating member 280, a force transmission member 230, and a holding mechanism 260.

The housing 210 is formed having a distal end side opening 212 and a proximal end side opening 211, and is a tubular member formed having a lumen 213 that communicates with the distal end side opening 212 and the proximal end side opening 211. The lumen 213 is a through-hole extending in the front-rear direction (Z-axis direction), and constitutes a portion of the lumen 13 of the medical connector 10. The housing 210, for example, is formed of a resin. A branched pipe portion 12 is formed on a distal end portion of the housing 210. Furthermore, as shown in FIG. 2, a proximal end portion of the housing 210 is inserted and fixed to the lumen 113 at the distal end portion of the housing 110 of the opening/closing mechanism 100. In the present embodiment, the movement toward the distal end side of the hemostasis valve 120 of the opening/closing mechanism 100 is regulated by the proximal end portion of the housing 210 of the fixing mechanism 200.

A cylindrical body accommodating space 215, which is a part of the lumen 213 having an increased diameter, is formed inside the housing 210 in the vicinity of the center in the front-rear direction. Furthermore, a retraction space 214 that communicates with the vicinity of the center of the cylindrical body accommodating space 215 in the front-rear direction is formed above the cylindrical body accommodating space 215 inside the housing 210. Moreover, a member accommodating space 216 is formed below the cylindrical body accommodating space 215, which communicates with the vicinity of the center of the cylindrical body accommodating space 215 in the front-rear direction, and extends in a diagonal direction toward the lower proximal end side and is open at the surface of the housing 210.

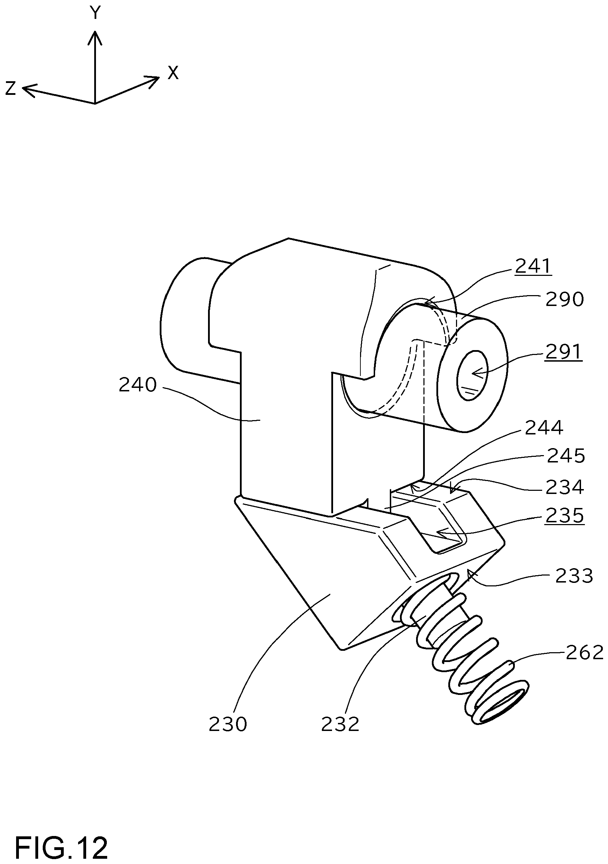

FIG. 12 is a perspective view showing an external configuration of the cylindrical body 290, the pressing member 240, and the force transmission member 230 of the first embodiment. The cylindrical body 290 is a flexible cylindrical member formed having a through-hole 291 into which a long medical device such as the guide wire GW is inserted. The cylindrical body 290 is formed, for example, of an elastic material such as silicone rubber. As shown in FIGS. 10 and 11, the cylindrical body 290 is attached inside the cylindrical body accommodating space 215 with a posture in which the axis is parallel to the front-rear direction (Z-axis direction). In the cylindrical body 290 attached to the housing 210, the through-hole 291 communicates with the distal end side opening 212 and the proximal end side opening 211 of the housing 210. Furthermore, the through-hole 291 and the lumen 213 of the housing 210 are coaxial with each other. The front-rear direction (Z-axis) direction is an example of a fifth direction in the claims.

As shown in FIGS. 10 and 11, substantially cylindrical stoppers 220 are attached to both ends of the cylindrical body 290. Each stopper 220 includes a main body portion 221 having substantially the same diameter as the cylindrical body 290, a first small diameter portion 223 that is positioned on the cylindrical body 290 side of the main body portion 221 and has a smaller diameter than the main body portion 221, and a second small diameter portion 222 that is positioned on the opposite side of the main body portion 221 to the cylindrical body 290 and has a smaller diameter than the main body portion 221. The first small diameter portion 223 of each stopper 220 is inserted into the through-hole 291 of the cylindrical body 290. Furthermore, the main body portion 221 of each stopper 220 is formed having a recess 224 to which a protrusion (not illustrated) of the housing 210 is fitted. As a result of the fitting between the protrusion and the recess 224, each stopper 220 is positioned in the front-rear direction with respect to the housing 210, and consequently, the cylindrical body 290 is positioned in the front-rear direction with respect to the housing 210.

As shown in FIG. 12, the pressing member 240 is a member formed having a through-hole 241 extending in the front-rear direction, and for example, is formed of a resin. The pressing member 240 has a substantially rectangular parallelepiped shape, and the length (dimension in the front-rear direction) of the part on the upper side of the center position of the through-hole 241 is longer than that of the other part. The inner diameter of the through-hole 241 is substantially the same as the outer diameter of the cylindrical body 290, and the cylindrical body 290 is inserted into the through-hole 241. Furthermore, a lower surface 244 of the pressing member 240 is a substantially flat surface that is substantially orthogonal to the up-down direction, and is formed having a guide protrusion 245 continuously extending in the front-rear direction.

As shown in FIGS. 10 and 11, the pressing member 240 is accommodated across the retraction space 214, the cylindrical body accommodating space 215, and the member accommodating space 216 of the housing 210. The pressing member 240 is capable of sliding along the up-down direction (Y-axis direction), which is a direction orthogonal to the axial direction (Z-axis direction) of the cylindrical body 290. The pressing member 240, which is capable of sliding in the up-down direction, can be positioned in a non-pressing position P5 shown in FIG. 10, in which the through-hole 241 becomes coaxial with the through-hole 291 of the cylindrical body 290, and a pressing position P6 shown in FIG. 11, which is upwardly displaced form the non-pressing position P5. When the pressing member 240 is positioned in the non-pressing position P5 (FIG. 10), the pressing member 240 substantially does not press the cylindrical body 290. Here, the state of substantially not pressing includes, in addition to a state where the inner peripheral surface of the through-hole 241 of the pressing member 240 and the outer peripheral surface of the cylindrical body 290 are separated, a state where the inner peripheral surface of the through-hole 241 of the pressing member 240 and the outer peripheral surface of the cylindrical body 290 are in close contact because the inner diameter of the through-hole 241 of the pressing member 240 is the same as, or slightly smaller than, the outer shape of the cylindrical body 290 before insertion into the through-hole 241. The up-down direction (Y-axis direction) is an example of a sixth direction in the claims, and the non-pressing position P5 is an example of a fifth position in the claims.

On the other hand, when the pressing member 240 is positioned in the pressing position P6 (FIG. 11), the pressing member 240 upwardly presses a part of the cylindrical body 290 excluding both end portions (a center part in the front-rear direction in the present embodiment) from the outer peripheral side, and causes the cylindrical body 290 to undergo elastic deformation. In this state, the inner peripheral surface of the elastically deformed cylindrical body 290 (primarily a contact position CP1, being a lower side part on the inner peripheral surface of the deformed part of the cylindrical body 290) is pressed against the long medical device, and because the long medical device is supported by shearing at a total of three positions, namely the contact position CP1, and contact portions CP2, being the upper part of the inner peripheral surface of both end portions of the cylindrical body 290 (or the stoppers 220), the long medical device is fixed by the fixing mechanism 200 and sliding is regulated in the front-rear direction. The retraction space 214 of the housing 210 has a sufficient area for the pressing member 240 to deform the cylindrical body 290 to an extent that allows the long medical device to be fixed. The pressing position P6 is an example of a sixth position in the claims.

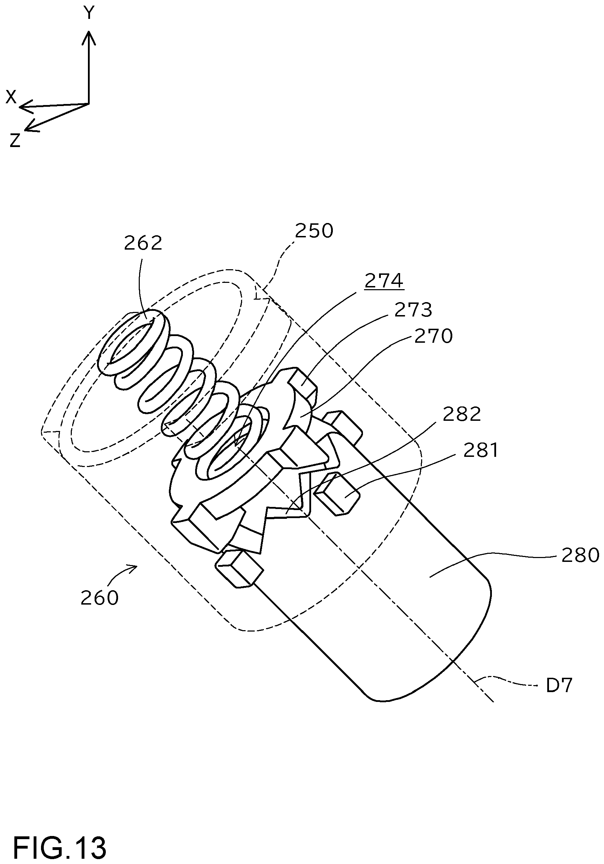

FIG. 13 is a perspective view showing an external configuration of the operating member 280 and the holding mechanism 260 in the first embodiment. As shown in FIGS. 10, 11 and 13, the operating member 280 is a substantially hollow cylindrical member extending in a direction (hereinafter referred to as "operation direction D7") not parallel to the front-rear direction (Z-axis direction). In the present embodiment, the operation direction D7 is a direction at an angle of 35 degrees or more and 55 degrees or less with respect to the front-rear direction, and for example, is a direction at an angle of 45 degrees with respect to the front-rear direction (a direction inclined at 45 degrees from the upper distal end side toward the lower proximal end side). The operating member 280 is supported by an outer cylinder 250 of the holding mechanism 260 described below, and is capable of sliding along the operation direction D7. A part of the operating member 280 on the proximal end side is exposed from the outer cylinder 250, and enables a pressing operation to be made by an operator such as a physician. The operating member 280, for example, is formed of a resin. The operation direction D7 is an example of a seventh direction in the claims.

As shown in FIG. 12, the force transmission member 230 is a substantially polygonal member, and for example, is formed of a resin. An upper surface 234 of the force transmission member 230 is a substantially flat surface that is substantially orthogonal to the up-down direction, and is formed having a guide groove 235 continuously extending in the front-rear direction. The guide protrusion 245 of the pressing member 240 is fitted into the guide groove 235. Furthermore, a surface 233 on the lower proximal end side of the force transmission member 230 is formed having a projection-shaped protrusion 232.

As shown in FIGS. 10 and 11, the force transmission member 230 is accommodated so as to be positioned between the pressing member 240 and the operating member 280 in the member accommodating space 216 of the housing 210, and is capable of sliding along the operation direction D7. The force transmission member 230 is capable of transmitting to the pressing member 240 a force along the operation direction D7 that causes the operating member 280 to approach the pressing member 240.

Because the force transmission member 230 has the configuration described above, it makes contact with the lower surface 244 of the pressing member 240, while also being configured to be capable of a relative sliding movement with respect to the pressing member 240 along the front-rear direction (Z-axis direction). That is, as shown in FIG. 10, when the pressing member 240 is in the non-pressing position P5 where it does not press the cylindrical body 290, the force transmission member 230 is in a retracted position P7 where it is retracted to the lower proximal end side. When the force transmission member 230 is in the retracted position P7, the force transmission member 230 does not press the pressing member 240. When the pressing member 240 is not being pressed by the force transmission member 230, the pressing member 240 is positioned in the non-pressing position P5 due to an elastic restoring force of the cylindrical body 290. The retracted position P7 is an example of a seventh position in the claims.

Furthermore, as shown in FIG. 11, when the force transmission member 230 slides diagonally upward from the retracted position P7 along the operation direction D7, and moves to an advanced position P8 closer to the cylindrical body 290 than the retracted position P7, the force transmission member 230 maintains a state where the guide protrusion 245 of the pressing member 240 is fitted in the guide groove 235 of the force transmission member 230, and performs a relative sliding movement with respect to the pressing member 240 toward the distal end side. When the force transmission member 230 is in the advanced position P8, the force transmission member 230 places the pressing member 240 in the pressing position P6 as a result of pressing the pressing member 240. The advanced position P8 is an example of an eighth position in the claims.

As shown in FIG. 13, the holding mechanism 260 adopts a so-called double knock mechanism, and switches states between a state in which the force transmission member 230 is held in the retracted position P7 and a state in which the force transmission member 230 is held in the advanced position P8 each time the operating member 280 slides along the operation direction D7. The holding mechanism 260 includes an outer cylinder 250, a rotor 270, and a biasing member 262. In the present embodiment, a portion of the operating member 280 constitutes a portion of the holding mechanism 260.

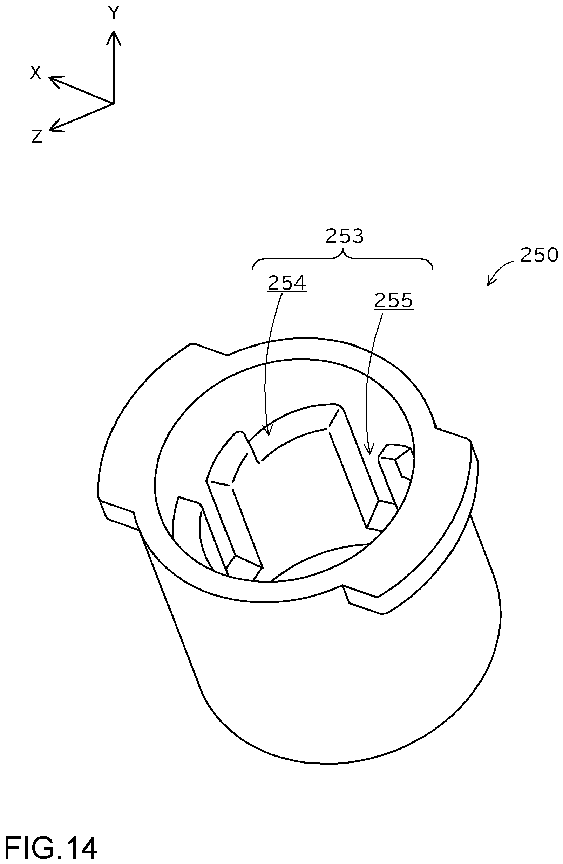

FIG. 14 is a perspective view showing an external configuration of the outer cylinder 250 of the first embodiment. As shown in FIGS. 13 and 14, the outer cylinder 250 is a substantially hollow cylindrical member, and for example, is formed of a resin. As shown in FIGS. 10 and 11, the outer cylinder 250 is attached to the distal end portion of a part of the housing 210 in which the member accommodating space 216 is formed, in a posture in which the axial direction coincides with the operation direction D7. As shown in FIG. 14, the inner peripheral surface of the outer cylinder 250 is formed having a tooth-shaped outer cylinder end surface cam 253. The outer cylinder end surface cam 253 is a cam formed having alternating shallow groove portions 254 and deep groove portions 255 in the circumferential direction. In the present embodiment, the outer cylinder end surface cam 253 includes four shallow groove portions 254 and four deep groove portions 255.

A tooth-shaped operating member end surface cam 282 is formed on the end surface on the upper distal end side of the substantially hollow cylindrical operating member 280. A plurality of (four) sliding contactors 281, which are substantially rectangular parallelepiped projections, are formed on the outer peripheral surface of the operating member 280. The operating member 280 is inserted into a hollow portion of the outer cylinder 250, and the sliding contactors 281 on the outer peripheral surface of the operating member 280 are fitted into the deep groove portions 255 on the inner peripheral surface of the outer cylinder 250. As a result, the operating member 280 is capable of sliding along the axial direction of the outer cylinder 250, that is, along the operation direction D7 in a state where rotation is restricted with respect to the outer cylinder 250.

The rotor 270 is a substantially circular plate-shaped member, and for example, is formed of a resin. The outer peripheral surface of the rotor 270 is formed having a plurality of (four) protrusions 273. The surface on the lower proximal end side of the protrusions 273 of the rotor 270 is formed having teeth. Here, the pitch of the peak parts of the operating member end surface cam 282 of the operating member 280 is offset by approximately half with respect to the pitch of the peak parts of the outer cylinder end surface cam 253 of the outer cylinder 250, and has a structure in which the protrusions 273 of the rotor 270 are unable to simultaneously engage with both the operating member end surface cam 282 and the outer cylinder end surface cam 253. Furthermore, the surface on the upper distal end side of the rotor 270 is formed having a recess 274.

The biasing member 262 is a substantially hollow cylindrical spring, and is formed of a metal such as stainless steel. The biasing member 262 is disposed between the rotor 270 and the force transmission member 230 (FIG. 12), and biases the force transmission member 230 in a direction toward the pressing member 240. One end portion of the biasing member 262 is inserted into the recess 274 of the rotor 270, and the protrusion 232 of the force transmission member 230 is inserted into a hollow portion of another end portion of the biasing member 262.

In a rotor-retracted state, in which the protrusions 273 of the rotor 270 are fitted in the deep groove portions 255 of the outer cylinder end surface cam 253 of the outer cylinder 250, the rotor 270 is positioned in a position that is retracted to the lower proximal end side with the deep groove portions 255 as a guide. Therefore, in the rotor-retracted state, as shown in FIG. 10, the force transmission member 230 is positioned in the retracted position P7 that is retracted to the lower proximal end side, and as a result, the pressing member 240 is positioned in the non-pressing position P5. The rotor-retracted state is maintained as long as the operating member 280 does not slide.

In the rotor-retracted state, when the operating member 280 advances along the operation direction D7 to the upper distal end side, the operating member end surface cam 282 engages the protrusions 273 of the rotor 270, which causes the rotor 270 to also advance with the operating member 280 in the same direction. When the rotor 270 advances to a position in which the protrusions 273 escape from the deep groove portions 255, the rotor 270 rotates in the circumferential direction by one-half of a peak due to the protrusions 273 sliding along the shape of the teeth of the operating member end surface cam 282. As a result, the protrusions 273 and the operating member end surface cam 282 are completely engaged with each other. Then, when the operating member 280 retracts along the operation direction D7 to the lower proximal end side, the rotor 270 also retracts in the same direction with the operating member 280. At this time, because the rotor 270 has already rotated by one-half of a peak, the protrusions 273 of the rotor 270 are fitted in the shallow groove portions 254 of the outer cylinder end surface cam 253 of the outer cylinder 250 rather than the deep groove portions 255. In a rotor-advanced state in which the protrusions 273 of the rotor 270 are fitted in the shallow groove portions 254, the rotor 270 is held in a position that is advanced to the upper distal end side compared to the rotor-retracted state in which the protrusions 273 are fitted in the deep groove portions 255. Therefore, in the rotor-advanced state, as shown in FIG. 11, the force transmission member 230 advances to the advanced position P8, and as a result, the pressing member 240 moves to the pressing position P6. The rotor-advanced state is maintained as long as the operating member 280 does not slide.

In the rotor-advanced state, when the operating member 280 advances along the operation direction D7 to the upper distal end side, the operating member end surface cam 282 engages the protrusions 273, which causes the rotor 270 to also advance with the operating member 280 in the same direction. When the rotor 270 advances to a position in which the protrusions 273 escape from the shallow groove portions 254, the rotor 270 rotates in the circumferential direction by one-half of a peak due to the protrusions 273 sliding along the shape of the teeth of the operating member end surface cam 282. As a result, the protrusions 273 and the operating member end surface cam 282 are completely engaged with each other. Then, when the operating member 280 retracts along the operation direction D7 to the lower proximal end side, the rotor 270 also retracts in the same direction with the operating member 280. At this time, because the rotor 270 has already rotated by one-half of a peak, the protrusions 273 of the rotor 270 are fitted in the deep groove portions 255 of the outer cylinder end surface cam 253 of the outer cylinder 250 rather than the shallow groove portions 254. As a result, the holding mechanism 260 returns to the rotor-retracted state, the force transmission member 230 returns to the retracted position P7, and the pressing member 240 moves to the non-pressing position P5. In this way, in the holding mechanism 260, the state of the holding mechanism 260 is switched between a state in which the force transmission member 230 is held in the retracted position P7 and a state in which the force transmission member 230 is held in the advanced position P8 each time the operating member 280 slides along the operation direction D7.

### A-6. Motion of Fixing Mechanism:

Next, the motion of the fixing mechanism 200 for a long medical device will be described. In the initial state, as shown in FIG. 10, the holding mechanism 260 is in the rotor-retracted state. In this state, the force transmission member 230 is positioned in the retracted position P7, and the pressing member 240 is not pressed by the force transmission member 230 and is positioned in the non-pressing position P5. Furthermore, because the cylindrical body 290 is not elastically deformed, a long medical device such as the guide wire GW that has been inserted into the lumen 213 of the housing 210 (the lumen 13 of the main pipe portion 11) is not fixed. That is, the fixing mechanism 200 is the released state fixing mechanism 200n.

For example, when the thumb of the operator that is gripping the medical connector 10 applies an operation (hereinafter, referred to as "fixing operation") of pressing the operating member 280 diagonally upward along the operation direction D7, the operating member 280 slides diagonally upward along the operation direction D7. Accordingly, the holding mechanism 260 switches from the rotor-retracted state to the rotor-advanced state. In this state, as shown in FIG. 11, the force transmission member 230 is positioned in the advanced position P8, and the pressing member 240 is pressed by the force transmission member 230 and is positioned in the pressing position P6. Moreover, the cylindrical body 290 is pressed by the pressing member 240 and undergoes elastic deformation, and the long medical device that has been inserted into the lumen 213 of the housing 210 is fixed. As a result, the fixing mechanism 200 becomes the fixed state fixing mechanism 200f.

Furthermore, when the fixing mechanism 200 is the fixed state fixing mechanism 200f, for example, when the thumb of the operator applies, in the same manner as the fixing operation, an operation (hereinafter, referred to as "unfixing operation") of pressing the operating member 280 along the operation direction D7 diagonally upward, the operating member 280 slides diagonally upward along the operation direction D7. Accordingly, the holding mechanism 260 switches from the rotor-advanced state to the rotor-retracted state. In this state, as shown in FIG. 10, the force transmission member 230 is positioned in the retracted position P7, and the pressing member 240 is not pressed by the force transmission member 230 and returns to the non-pressing position P5 due to the elastic restoring force of the cylindrical body 290. Moreover, the cylindrical body 290 is not pressed by the pressing member 240 and does not undergo elastic deformation, and the fixing of the long medical device that has been inserted into the lumen 213 of the housing 210 is released. As a result, the fixing mechanism 200 returns to the released state fixing mechanism 200n.

In this way, the fixing mechanism 200 switches states to the released state fixing mechanism 200n or the fixed state fixing mechanism 200f each time the pressing operation (the fixing operation and the unfixing operation) is performed with respect to the operating member 280.

### A-7. Technical Effects of Fixing Mechanism:

As described above, in the fixing mechanism 200 of the present embodiment, the holding mechanism 260 switches between a state in which the force transmission member 230 is held in the retracted position P7 and a state in which the force transmission member 230 is held in the advanced position P8 each time the operating member 280 slides along the operation direction D7. Therefore, it is possible to achieve fixing and unfixing of the long medical device with a simple operation in which the operator presses the operating member 280 with the thumb while gripping the medical connector 10, and because the degree to which the long medical device is fixed can be grasped visually or by feel, the operability of the fixing mechanism 200 can be improved.

The holding mechanism 260 includes the cylindrical biasing member 262 that biases the force transmission member 230 in a direction that approaches the pressing member 240, and the surface facing the biasing member 262 of the force transmission member 230 is formed having a protrusion 232 that is inserted into the hollow portion of the biasing member 262. According to fixing mechanism 200 of the present embodiment, it is possible to easily and accurately perform the positioning between the force transmission member 230 and the biasing member 262, and it is also possible to effectively transmit the biasing force of the biasing member 262 to the force transmission member 230, which enables the accuracy of the motion of the fixing mechanism 200 to be improved.

The angle formed between the direction parallel to the axis of the cylindrical body 290 (Z-axis direction) and the operation direction D7 of the operating member 280 is 35 degrees or more and 55 degrees or less. As a result, according to the fixing mechanism 200 of the present embodiment, it is possible for an operator to very easily operate the operating member 280 with the thumb while gripping the medical connector 10, and the operability of the fixing mechanism 200 can be effectively improved.

The force transmission member 230 makes contact with the surface of the pressing member 240, while also being configured to be capable of a relative sliding movement with respect to the pressing member 240 along a direction (Z-axis direction) parallel to the axis of the cylindrical body 290. Therefore, according to the fixing mechanism 200 of the present embodiment, the sliding of the force transmission member 230 along the operation direction D7 and the sliding of the pressing member 240 along the up-down direction can be efficiently converted, and the operability of the fixing mechanism 200 can be effectively improved.

### B. Second embodiment:

### B-1. Configuration of Medical Connector:

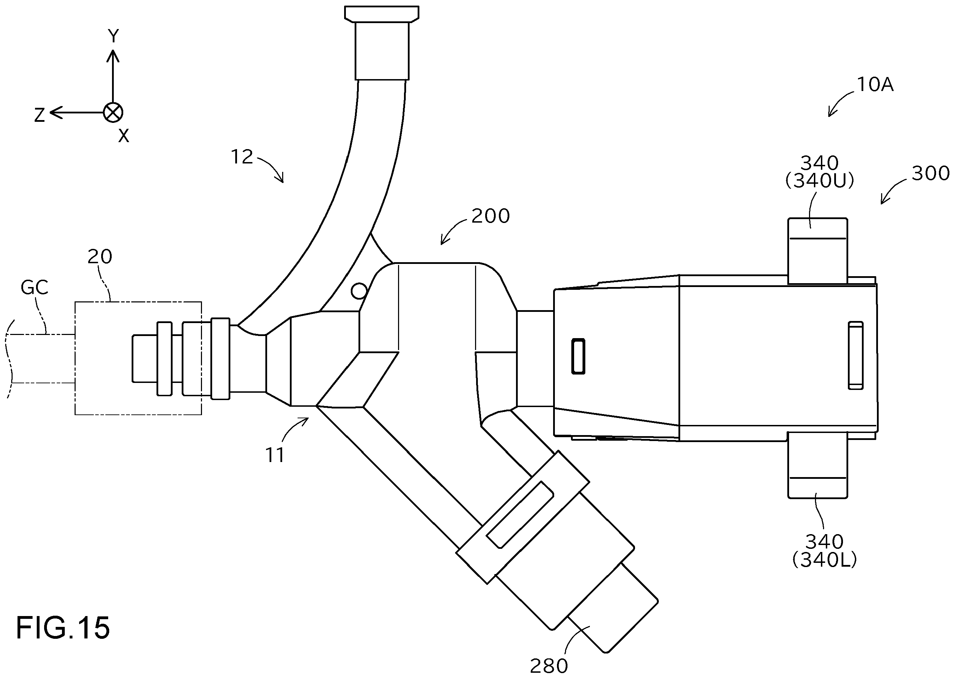

FIG. 15 is an explanatory diagram showing an external configuration of a medical connector 10A in a second embodiment. Hereinafter, those components of the medical connector 10A of the second embodiment that are the same components as those of the medical connector 10 of the first embodiment described above are denoted by the same reference numerals, and the description thereof is omitted as appropriate.

The medical connector 10A of the second embodiment includes an opening/closing mechanism 300 and a fixing mechanism 200. The configuration of the fixing mechanism 200 included in the medical connector 10A of the second embodiment is the same as the configuration of the fixing mechanism 200 included in the medical connector 10 of the first embodiment. Therefore, the opening/closing mechanism 300 included in the medical connector 10A of the second embodiment will be described below.

### B-2. Configuration of Opening/Closing Mechanism:

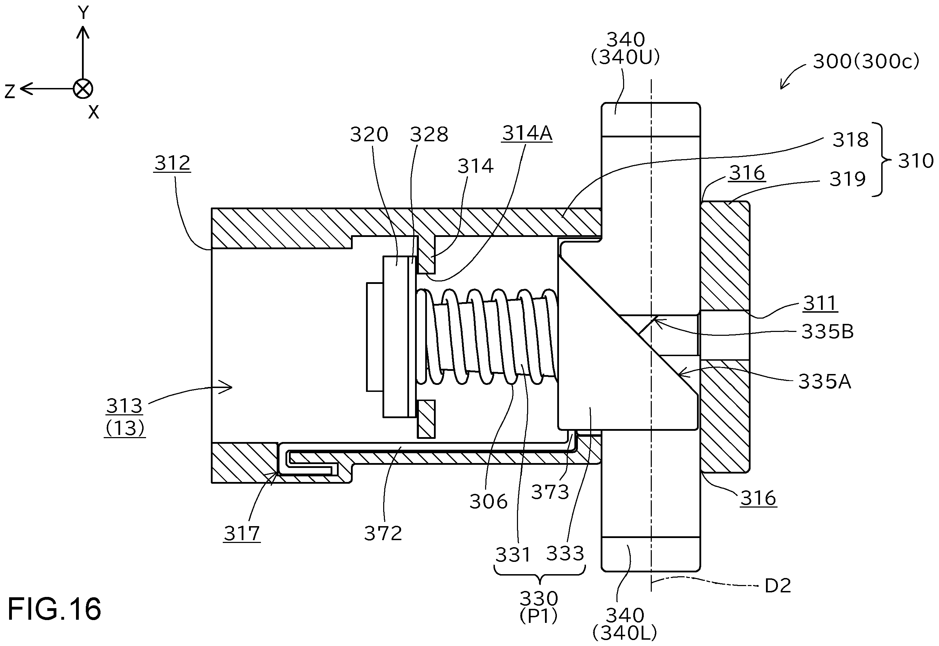

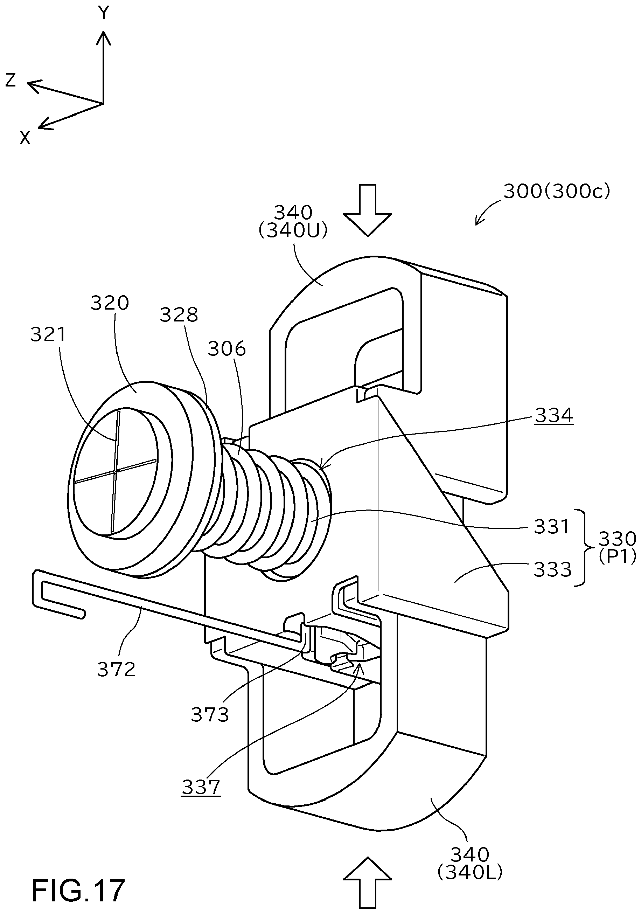

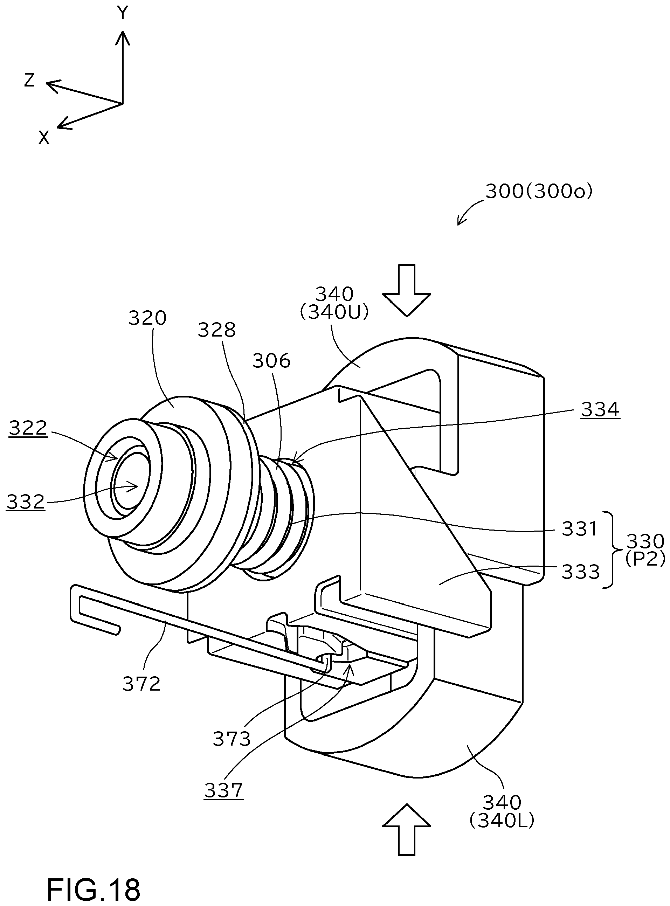

FIGS. 16 to 18 are explanatory diagrams showing a configuration of the opening/closing mechanism 300 of the second embodiment, where FIG. 16 shows the configuration (for some members, a configuration of the side surface) of a longitudinal cross-section (YZ cross-section), and FIGS. 17 and 18 show a cross-sectional perspective view (for some members, an external perspective view). In FIGS. 17 and 18, illustration of the housing 310 has been omitted. Furthermore, FIGS. 16 and 17 show the opening/closing mechanism 300 in a state in which the hemostasis valve 320 is closed (hereinafter referred to as "closed state opening/closing mechanism 300c"), and FIG. 18 shows the opening/closing mechanism 300 in a state in which the hemostasis valve 320 is open (hereinafter referred to as "open state opening/closing mechanism 300o).

The opening/closing mechanism 300 is a mechanism for opening and closing the hemostasis valve 320, which suppresses the outflow of blood via the lumen 13 of the main pipe portion 11 (FIG. 15) of the medical connector 10A. The opening/closing mechanism 300 of the present embodiment is a mechanism that switches between a state in which the hemostasis valve 320 is closed or a state in which the hemostasis valve 320 is open state each time the operator performs a pressing operation with respect to the operating member 340. The opening/closing mechanism 300 includes a housing 310, a hemostasis valve 320, a through-member 330, an operating member 340, a biasing member 306, and a pin 372.

As shown in FIG. 16, the housing 310 is formed having a distal end side opening 312 and a proximal end side opening 311, and is a tubular member formed having a lumen 313 that communicates with the distal end side opening 312 and the proximal end side opening 311. More specifically, the housing 310 includes a substantially tubular distal end side member 318, and a lid-shaped proximal end side member 319 that closes the opening on the proximal end side of the distal end side member 318, such that an opening formed in the distal end of the distal end side member 318 constitutes the distal end side opening 312, and a through-hole formed in the proximal end side member 319 constitutes the proximal end side opening 311. The lumen 313 is a through-hole extending in the front-rear direction (Z-axis direction), and constitutes a portion of the lumen 13 of the main pipe portion 11 of the medical connector 10A. The housing 310, for example, is formed of a resin. The Z-axis direction is an example of a first direction in the claims.

In the vicinity of the center of the inside of the housing 310 in the front-rear direction, a substantially flat plate-shaped partition wall 314 that is substantially orthogonal to the front-rear direction is formed. The partition wall 314 is formed having a through-hole 314A that passes through the partition wall 314 in the front-rear direction and constitutes a portion of the lumen 313. The transverse cross-sectional shape of the through-hole 314A, for example, is substantially circular. Also, a groove 317 for accommodating the pin 372 is formed in a lower part of the inside of the housing 310. Furthermore, a pair of upper and lower through-holes 316 for the operating member is formed in the proximal end portion of the housing 310.

The hemostasis valve 320 is a member having the same configuration as the hemostasis valve 120 of the first embodiment. The hemostasis valve 320 is fixed at a position further toward the distal end side than the partition wall 314 inside the housing 310. The hemostasis valve 320 is normally in the closed state in which the slit 321 is closed and the valve is closed (FIGS. 16 and 17). Furthermore, when the hemostasis valve 320 is pressed from the proximal end side, the hemostasis valve 320 switches to the open state, in which a through-hole 322 is formed that passes through the hemostasis valve 320 in front-rear direction (FIG. 18). When the pressing force from the proximal end side is no longer applied, the hemostasis valve 320 elastically deformed and returns to the closed state. In the present embodiment, a substantially annular boss 328 is disposed between the hemostasis valve 320 and the partition wall 314 of the housing 310.

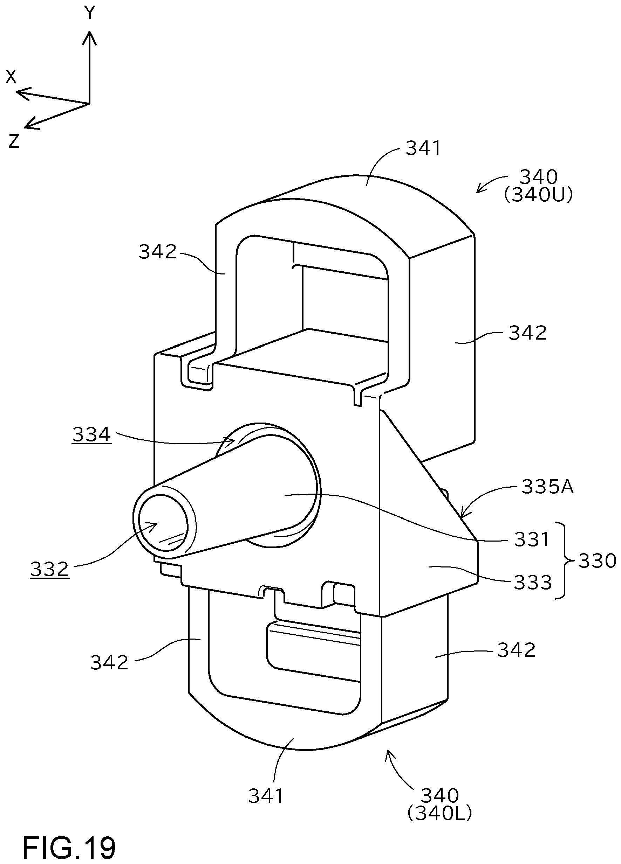

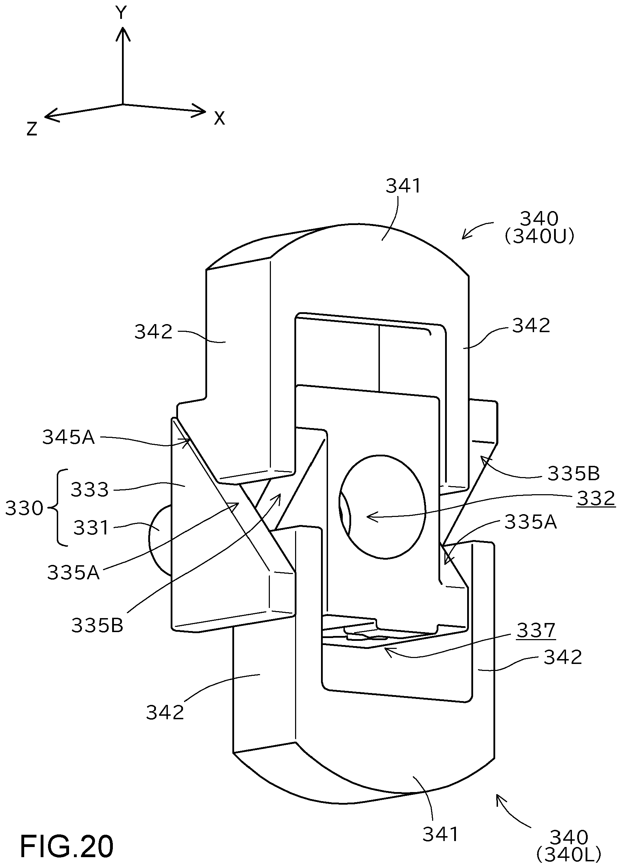

FIGS. 19 and 20 are perspective views showing an external configuration of the through-member 330 and the operating member 340 according to the second embodiment. The through-member 330 is a member formed having a through-hole 332 extending in the front-rear direction, and for example, is formed of a resin. More specifically, the through-member 330 includes a substantially cylindrical main body portion 331 formed having a distal end side part of the through-hole 332, and a substantially rectangular parallelopiped-shaped connection portion 333 that is connected to the proximal end side of the main body portion 331 and formed having a proximal end side part of the through-hole 332. The distal end side surface of the connection portion 333 is formed having a recess 334 that surrounds the outer periphery of the main body portion 331. Furthermore, as shown in FIGS. 17, 18, and 20, the lower surface of the connection portion 333 is formed having a motion restricting groove 337. The motion restricting groove 337 is a so-called heart cam groove, and is a groove having a heart shape that is connected over the entire circumference.

The proximal end side surface of the connection portion 333 is formed having four through-member side contact surfaces 335 that are not parallel to the front-rear direction (Z-axis direction) and the up-down direction (Y-axis direction), and parallel to the left-right direction (X-axis direction). More specifically, the proximal end side surface of the connection portion 333 is formed having a pair of through-member side first contact surfaces 335A facing the upper proximal end side, and a pair of through-member side second contact surfaces 335B facing the lower proximal end side. The pair of through-member side first contact surfaces 335A is disposed so as to sandwich the through-hole 332 and face each other in the left-right direction, and similarly, the pair of through-member side second contact surfaces 335B is disposed so as to sandwich the through-hole 332 and face each other in the left-right direction. The inclination angle of the through-member side contact surfaces 335 with respect to the front-rear direction is, for example, 45 degrees. The up-down direction is an example of a second direction in the claims, and the left-right direction is an example of a fourth direction in the claims.

As shown in FIG. 16, the through-member 330 is accommodated inside the housing 310 further toward the proximal end side than the hemostasis valve 320. In the through-member 330, the through-hole 332 communicates with the proximal end side opening 311 of the housing 310. Furthermore, the through-hole 332 and the lumen 313 of the housing 310 are coaxial with each other.

The through-member 330 accommodated in the housing 310 is capable of sliding in the front-rear direction in a state in which the position in the up-down direction and the left-right direction with respect to the housing 310 is fixed. The through-member 330 is positioned such that the main body portion 331 of the through-member 330 faces the through-hole 314A of the partition wall 314 in the front-rear direction. The through-member 330, which is capable of sliding in the front-rear direction, has the distal end portion of the main body portion 331 inserted into the through-hole 314A, and can be positioned in a pressing position P2 that presses the hemostasis valve 320 and places the hemostasis valve 320 in the open state, and a non-pressing position P1 that does not press the hemostasis valve 320 and places the hemostasis valve 320 in the closed state. As shown in FIG. 18, in the state in which the through-member 330 is positioned in the pressing position P2, the through-hole 322 formed in the hemostasis valve 320 and the through-hole 332 of the through-member 330 communicate with each other. The distal end side of the through-member 330 is capable of moving to a position in which the connection portion 333 makes contact with the partition wall 314 of the housing 310, and the proximal end side is capable of moving to a position in which the proximal end of the through-member 330 makes contact with the housing 310. The pressing position P2 is an example of a second position in the claims, and the non-pressing position P1 is an example of a first position in the claims.

The biasing member 306 is a member having the same configuration as the biasing member 106 of the first embodiment, and biases the through-member 330 toward the proximal end side. As shown in FIG. 16, the biasing member 306 is accommodated in a position between the hemostasis valve 320 and the through-member 330 in the housing 310. The main body portion 331 of the through-member 330 is inserted into a hollow portion of the biasing member 306 from the proximal end side, and the proximal end portion of the biasing member 306 is accommodated in the recess 334 formed in the connection portion 333 of the through-member 330. The through-member 330, which is biased toward the proximal end side by the biasing member 306, is positioned in the non-pressing position P1 (FIGS. 16 and 17) that does not press the hemostasis valve 320 when the through-member 330 is not receiving a force from the operating member 340 and the pin 372.

The pin 372 is a member having the same configuration as the pin 172 of the first embodiment. The pin 372 is attached to the housing 310 with a posture that extends in the front-rear direction. The end portion of the pin 372 on the distal end side is accommodated in the groove 317 formed in the housing 310 and is fixed. The end portion 373 of the pin 372 on the proximal end side is upwardly bent and loosely fitted into the motion restricting groove 337 that is formed in the connection portion 333 of the through-member 330. The position of the end portion 373 of the pin 372 in the motion restricting groove 337 changes with the sliding of the through-member 330 in the front-rear direction (Z-axis direction).

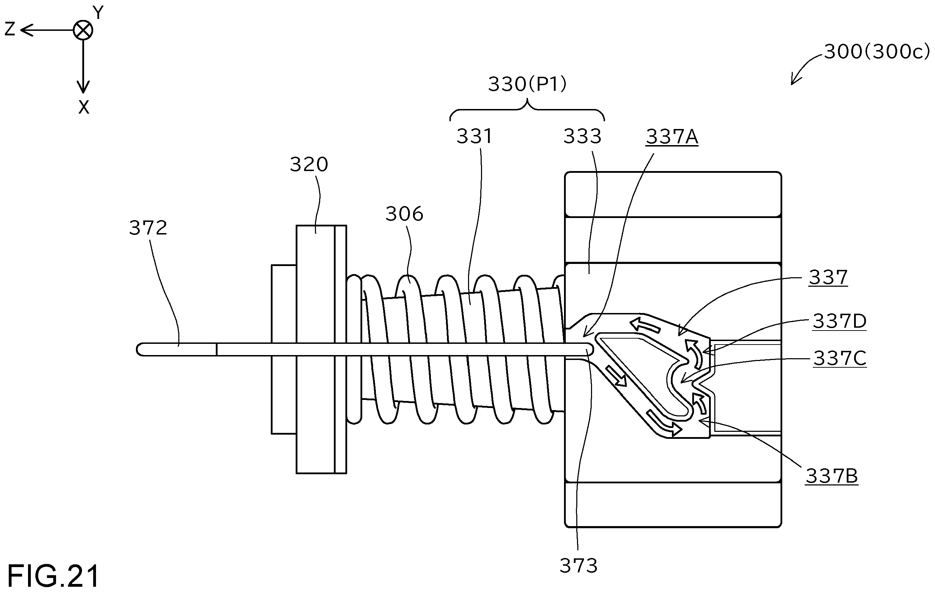

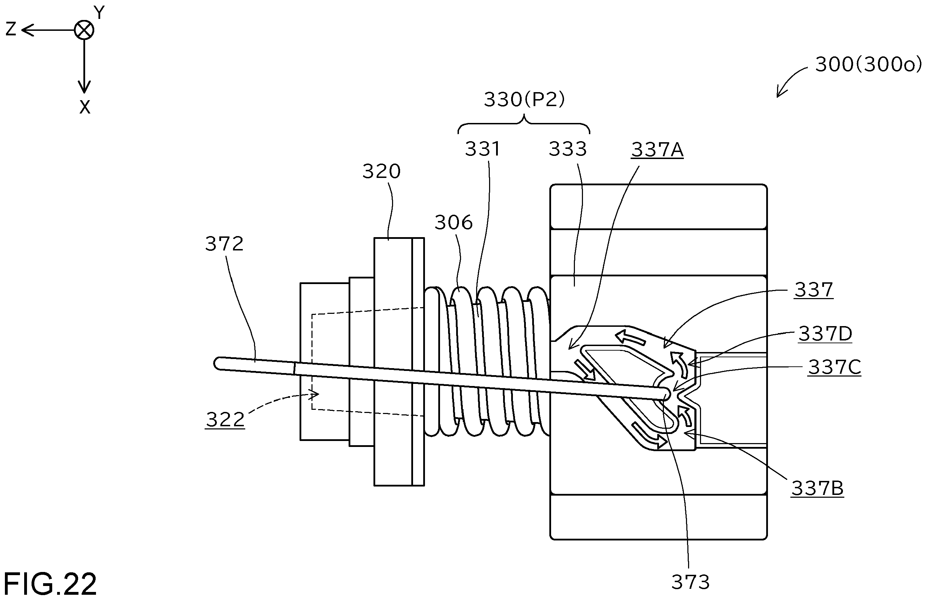

FIGS. 21 and 22 are explanatory diagrams showing the position of the pin 372 in the motion restricting groove 337 of the second embodiment. FIGS. 21 and 22 show a lower surface configuration of the pin 372, the through-member 330, and the hemostasis valve 320. FIG. 21 shows the closed state opening/closing mechanism 300c in which the through-member 330 is in the non-pressing position P1 and the hemostasis valve 320 is closed, and FIG. 22 shows the open state opening/closing mechanism 300o in which the through-member 330 is in the pressing position P2 and the through-hole 322 is formed in the hemostasis valve 320.

As indicated by the white arrows in FIGS. 21 and 22, as the through-member 330 slides in the front-rear direction (Z-axis direction), the end portion 373 of the pin 372 swings in the left-right direction (X-axis direction), and relatively moves in one direction (counterclockwise in the illustrated example) in the motion restricting groove 337. That is to say, as shown in FIG. 21, in the first state in which the through-member 330 is in the non-pressing position P1, the end portion 373 is positioned at a bottom portion (hereinafter, referred to as "closed position portion 337A") of the heart shape of the motion restricting groove 337. In the first state, the movement of the through-member 330 in the front-rear direction is not restricted by the pin 372. When the through-member 330 moves toward the distal end side (positive Z-axis direction) from the first state, the end portion 373 also relatively moves in the motion restricting groove 337 toward the proximal end side. In the second state, in which the through-member 330 is displaced from the non-pressing position P1 to the pressing position P2, and reaches a position further toward the distal end side than the pressing position P2, the end portion 373 is positioned at the first top portion 337B, being one of a pair of top portions that sandwich the heart-shaped valley portion of the motion restricting groove 337. In this state, the movement of the through-member 330 toward the proximal end side is not restricted by the pin 372. In the third state, in which the through-member 330 has moved to the pressing position P2 by slightly returning toward the proximal end side from the second state, as shown in FIG. 22, the end portion 373 of the pin 372 relatively moves toward the distal end side in the motion restricting groove 337, and reaches the heart-shaped valley portion (hereinafter, referred to as "open holding portion 337C") of the motion restricting groove 337. In the third state, the movement of the through-member 330 to the proximal end side is restricted due to the interference between the pin 372 and the motion restricting groove 337, and a state in which the through-member 330 is positioned in the pressing position P2 is maintained. In the fourth state, in which the through-member 330 reaches a position further toward the distal end side than the pressing position P2 by moving slightly toward the distal end side from the third state, the end portion 373 relatively moves toward the proximal end side in the motion restricting groove 337, and is positioned at the other of the pair of top portions (hereinafter, referred to as "second top portion 337D") that sandwich the heart-shaped valley portion of the motion restricting groove 337. In the fourth state, the movement of the through-member 330 toward the proximal end side is not restricted by the pin 372. When the through-member 330 moves toward the proximal end side to the non-pressing position P1 from the fourth state, the end portion 373 also relatively moves toward the distal end side in the motion restricting groove 337 and returns to the closed position portion 337A of the motion restricting groove 337 (FIG. 21).

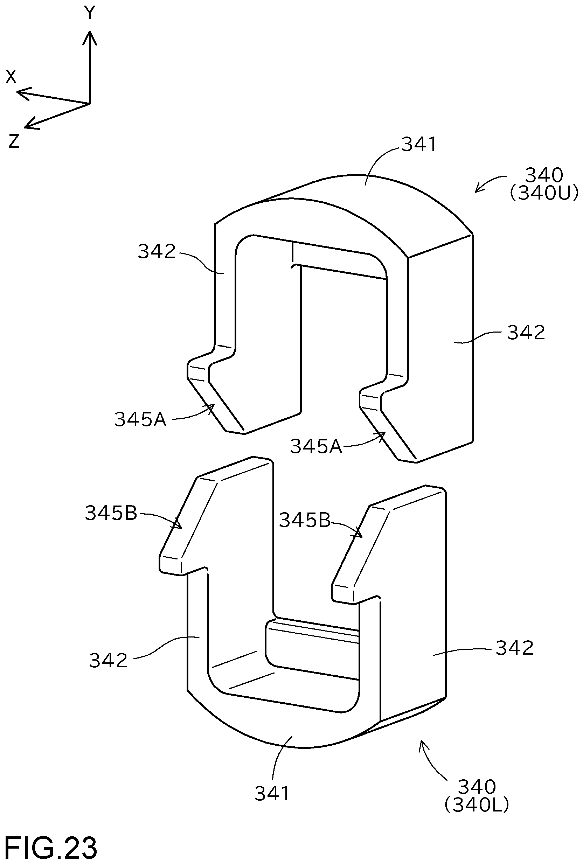

FIG. 23 is an external perspective view showing a configuration of the operating member 340 of the second embodiment. As shown in FIGS. 16 to 20, and 23, the operating member 340 has a first piece 340U and a second piece 340L that face each other in the up-down direction. The second piece 340L is disposed below the first piece 340U. The first piece 340U and the second piece 340L are accommodated in the housing 310 so as to be capable of sliding in a direction (hereinafter, referred to as "operation direction D2") that is not parallel to the front-rear direction (Z-axis direction). In the present embodiment, the operation direction D2 is an up-down direction that forms an angle of substantially 90 degrees with the front-rear direction. Herein, substantially 90 degrees refers to a range of 90 degrees plus or minus about 5 degrees. The operation direction D2 is an example of a second direction in the claims.

The first piece 340U and the second piece 340L that constitute the operating member 340 each have a pair of arm portions 342 extending in the up-down direction, and a connection part 341 that joins the end portions of the pair of arm portions 342 to each other. As shown in FIG. 16, the connection part 341 of each of the first piece 340U and the second piece 340L is exposed from the housing 310 via a through-hole 316 for the operating member, which allows a pressing operation to be performed by an operator such as a physician. Furthermore, the distal end portion of the arm portions 342 of each of the first piece 340U and the second piece 340L is formed having an operating member side contact surface 345 that is not parallel to the front-rear direction (Z-axis direction) and the up-down direction (Y-axis direction), and is parallel to the left-right direction (X-axis direction). More specifically, the distal end portion of the arm portions 342 of the first piece 340U is formed having operating member side first contact surfaces 345A that face the lower distal end side. The operating member side first contact surfaces 345A are surface that makes contact with each of the pair of through-member side first contact surfaces 335A of the through-member 330. Moreover, the distal end portion of the arm portions 342 of the second piece 340L is formed having operating member side second contact surfaces 345B that face the upper distal end side. The operating member side second contact surfaces 345B are surfaces that make contact with each of the pair of through-member side second contact surfaces 335B of the through-member 330. The inclination angle of the operating member side contact surfaces 345 with respect to the front-rear direction is, for example, 45 degrees.

As shown in FIGS. 16 and 17, when the through-member 330 is in the non-pressing position P1 that does not press the hemostasis valve 320, the first piece 340U and the second piece 340L that constitute the operating member 340 are in a state in which they are far away from each other along the up-down direction (hereinafter, referred to as "separated state"). When the first piece 340U and the second piece 340L slide along the up-down direction so as to approach each other (that is, toward the inside of the housing 310), the operating member side first contact surfaces 345A of the first piece 340U and the through-member side first contact surfaces 335A of the through-member 330 make contact while undergoing a relative sliding movement, and the operating member side second contact surfaces 345B of the second piece 340L and the through-member side second contact surfaces 335B of the through-member 330 make contact while undergoing a relative sliding movement, which causes the through-member 330 to be pressed toward the distal end side and displaced toward the distal end side (FIG. 18). The sliding range of the operating member 340 is set to a range that enables the through-member 330 to be moved further toward the distal end side than the pressing position P2.

### B-3. Motion of Opening/Closing Mechanism:

Next, the motion of the opening/closing mechanism 300 for the hemostasis valve 320 will be described. In the initial state, as shown in FIGS. 16 and 17, the through-member 330 is positioned in the non-pressing position P1, in which the through-member 330 receives a biasing force from the biasing member 306 and does not press the hemostasis valve 320, and the first piece 340U and the second piece 340L that constitute the operating member 340 indirectly receive the biasing force via the through-member 330 and are in a state where they are separated from each other in the up-down direction. In this state, the hemostasis valve 320 is closed, and the opening/closing mechanism 300 is the closed state opening/closing mechanism 300c. At this time, as shown in FIG. 21, the end portion 373 of the pin 372 is positioned in the closed position portion 337A of the motion restricting groove 337 in the through-member 330.

For example, when the thumb and index finger of the operator that are gripping the medical connector 10A apply an operation (hereinafter, referred to as "valve-opening operation") of pressing the first piece 340U and the second piece 340L constituting the operating member 340 toward the inner side of the housing 310, the through-member 330 is pressed by the first piece 340U and the second piece 340L and moves toward the distal end side. Furthermore, the end portion 373 of the pin 372 relatively moves toward the proximal end side in the motion restricting groove 337. When the through-member 330 moves a fixed distance or more toward the distal end side, the distal end portion of the main body portion 331 of the through-member 330 presses the hemostasis valve 320 and places the hemostasis valve 320 in the open state. When the valve-opening operation is released by the operator after the through-member 330 reaches a position further toward the distal end side than the pressing position P2 and the end portion 373 of the pin 372 reaches the first top portion 337B of the motion restricting groove 337, as shown in FIG. 22, the through-member 330 returns slightly toward the proximal end side due to the biasing force of the biasing member 306, and the end portion 373 also relatively moves toward the distal end side in the motion restricting groove 337 and reaches the open holding portion 337C. In this state, because the movement of the through-member 330 toward the proximal end side is restricted due to the interference between the pin 372 and the motion restricting groove 337, a state in which the through-member 330 is positioned in the pressing position P2 is maintained. As a result, the opening/closing mechanism 300 becomes the open state opening/closing mechanism 300o shown in FIG. 18.

In addition, when the opening/closing mechanism 300 is the open state opening/closing mechanism 300o, for example, when the thumb and index finger of the operator apply an operation (hereinafter, referred to as "valve-closing operation") of pressing the first piece 340U and the second piece 340L constituting the operating member 340 along the up-down direction toward the inner side of the housing 310 in the same manner as the valve-opening operation, the through-member 330 is pressed by the first piece 340U and the second piece 340L constituting the operating member 340 and moves to a position further toward the distal end side than the pressing position P2. Furthermore, the end portion 373 of the pin 372 relatively moves toward the proximal end side and reaches the second top portion 337D from the open holding portion 337C in the motion restricting groove 337. In this state, the movement of the through-member 330 toward the proximal end side is not restricted by the pin 372. As a result, when the valve-closing operation is released by the operator, the through-member 330 moves to the proximal end side due to the biasing force of the biasing member 306, and returns to the non-pressing position P1. Accordingly, as shown in FIG. 21, the end portion 373 of the pin 372 relatively moves toward the distal end side in the motion restricting groove 337 and returns to the closed position portion 337A. As a result, the hemostasis valve 320 is placed in the closed state, and the opening/closing mechanism 300 becomes the closed state opening/closing mechanism 300c shown in FIGS. 16 and 17.

In this way, the opening/closing mechanism 300 switches states between the closed state opening/closing mechanism 300c and the open state opening/closing mechanism 300o each time the pressing operation (the valve-opening operation and the valve-closing operation) is performed with respect to the first piece 340U and the second piece 340L constituting the operating member 340.

### B-4. Technical Effects of Opening/Closing Mechanism:

As described above, because the opening/closing mechanism 300 of the second embodiment has the same configuration as the first embodiment described above, like the first embodiment, it is possible for an operator to very easily perform the opening and closing operation of the hemostasis valve 320 with the thumb and/or index finger while gripping the medical connector 10A, and the operability of the opening/closing mechanism 300 can be improved.

The biasing member 306 is cylindrical, and the main body portion 331 serving as a protrusion that is inserted into the hollow portion of the biasing member 306 is formed on the surface of the through-member 330 facing the biasing member 306. Therefore, according to the opening/closing mechanism 300 of the second embodiment, it is possible to easily and accurately perform the positioning between the through-member 330 and the biasing member 306, and it is also possible to effectively transmit the biasing force of the biasing member 306 to the through-member 330, which enables the accuracy of the motion of the opening/closing mechanism 300 to be improved.

The angle formed between the extending direction (Z-axis direction) of the lumen 313 of the housing 310 and the operation direction D2 of the operating member 340 is substantially 90 degrees. Therefore, according to the opening/closing mechanism 300 of the second embodiment, it is possible for an operator to very easily perform the opening and closing operation of the hemostasis valve 320 with the thumb and/or index finger while gripping the medical connector 10A, and the operability of the opening/closing mechanism 300 can be effectively improved.

The operating member 340 is formed of the first piece 340U and the second piece 340L that face each other in the up-down direction (Y-axis direction), and the through-member 330 is pressed and displaced toward the distal end side as a result of the first piece 340U and the second piece 340L sliding along the up-down direction so as to approach each other. Therefore, according to the opening/closing mechanism 300, for a hemostasis valve, it is possible for an operator to very easily and stably perform the opening and closing operation of the hemostasis valve 320 by performing the operation of pinching the first piece 340U and the second piece 340L between the thumb and index finger while gripping the medical connector 10A, and the operability of the opening/closing mechanism 300 can be effectively improved.

The first piece 340U and the second piece 340L each have an operating member side contact surface 345, being a surface that is parallel to the left-right direction (X-axis direction) which is orthogonal to both the extending direction (Z-axis direction) of the lumen 313 of the housing 310 and the up-down direction (Y-axis direction), that is not parallel to both the extending direction and the up-down direction. Furthermore, the through-member 330 has through-member side first contact surfaces 335A that make contact with the operating member side contact surface 345 of the first piece 340U, and through-member side second contact surfaces 335B that make contact with the operating member side contact surface 345 of the second piece 340L, the through-member side first contact surfaces 335A and the through-member side second contact surfaces 335B being surfaces that are parallel to the left-right direction and not parallel to both the extending direction of the lumen 313 and the up-down direction. Therefore, according to the opening/closing mechanism 300 of the present embodiment, the sliding of the operating member 340 along the up-down direction substantially orthogonal to the extending direction of the lumen 313 of the housing 310 and the sliding of the through-member 330 in the extending direction can be efficiently converted, and the operability of the opening/closing mechanism 300 can be effectively improved.

### C. Third Embodiment:

### C-1. Configuration of Medical Connector:

FIG. 24 is an explanatory diagram illustrating a longitudinal sectional configuration (YZ section) of a medical connector 10B according to a third embodiment. Hereinafter, those components of the medical connector 10B that are the same components as those of the medical connector 10 of the first embodiment described above are denoted by the same reference numerals and the description thereof is omitted as appropriate.

The medical connector 10B includes an opening/closing mechanism 100 and a fixing mechanism 200B. Because the opening/closing mechanism 100 included in the medical connector 10B is the same as the opening/closing mechanism 100 included in the medical connector 10 of the first embodiment, the description will be omitted, while the fixing mechanism 200B which is different from that of the first embodiment will be described as follows.

### C-2. Configuration of Fixing Mechanism:

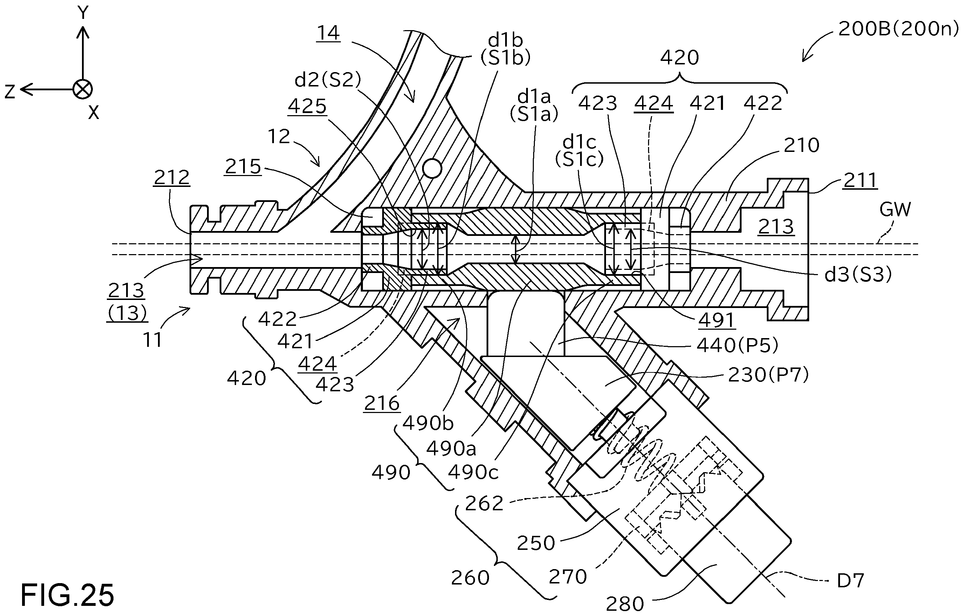

FIGS. 25 and 26 are explanatory diagrams illustrating the configuration of the fixing mechanism 200B, and the configuration in a longitudinal cross-section (YZ cross-section) is illustrated. FIG. 25 shows the fixing mechanism 200B in a state in which the fixing of a long medical device such as the guide wire GW has been released (released state fixing mechanism 200n), and FIG. 26 shows the fixing mechanism 200B in a state in which a long medical device has been fixed (fixed state fixing mechanism 200f). Note that some of the members are illustrated as a side view.

The fixing mechanism 200B of the third embodiment includes a cylindrical body 490 instead of the cylindrical body 290 of the fixing mechanism 200 of the first embodiment. The cylindrical body 490 is different from the cylindrical body 290 in the configuration of the through-hole 491. That is, the inner diameter of the through-hole 491 passing through the cylindrical body 490 in the longitudinal direction changes according to the position along the axial direction. More specifically, in the cylindrical body 490, the inner diameter d1a of the through-hole 491 at a center portion 490a is smaller than the inner diameter dib at a distal end portion 490b, and smaller than the inner diameter d1c at a proximal end portion 490c. In other words, the through-hole 491 has a larger diameter at the distal end portion and the proximal end portion than the center portion. Therefore, the area of the through-hole 491 in the transverse cross-section of the fixing mechanism 200B has the area S1a at the center portion 490a smaller than the area S1b at the distal end portion 490b and the area S1c at the proximal end portion 490c. The shape of the through-hole 491 in the transverse cross-section at each position may be any shape, such as a circular shape. The through-hole 491 is an example of a first through-hole in the claims.

Like the first embodiment, stoppers 420 are attached to the distal end portion and the proximal end portion of the cylindrical body 490. Each stopper 420 includes a main body portion 421 having substantially the same outer diameter as the center portion 490a of the cylindrical body 490, a first small diameter portion 423 that is positioned on the cylindrical body 490 side of the main body portion 421 and has a smaller outer diameter than the main body portion 421, and a second small diameter portion 422 that is positioned on the opposite side of the main body portion 421 to the cylindrical body 490 and has a smaller outer diameter than the main body portion 421. The first small diameter portion 423 is inserted into the through-hole 491. Furthermore, the main body portion 421 is formed having a recess 424 to which a protrusion (not illustrated) of the housing 210 is fitted. As a result of the fitting between the protrusion and the recess 424, the stoppers 420 are positioned in the front-rear direction with respect to the housing 210, and consequently, the cylindrical body 490 is positioned in the front-rear direction with respect to the housing 210. In other words, the stopper 420 on the distal end side connects the distal end portion 490b to the housing 210, and the stopper 420 on the proximal end side connects the proximal end portion 490c to the housing 210. Each stopper 420 is formed having a through-hole 425 that communicates with the through-hole 491. The shape of the through-hole 425 in the transverse cross-section at each portion may be any shape, such as a circular shape. The stopper 420 on the distal end side is an example of a distal end side connection portion in the claims, and the through-hole 425 of the stopper 420 on the distal end side is an example of a second through-hole in the claims. Furthermore, the stopper 420 on the proximal end side is an example of a proximal end side connection portion in the claims, and the through-hole 425 of the stopper 420 on the proximal end side is an example of a third through-hole in the claims.

In the first small diameter portion 423 of the stopper 420 on the distal end side (the part inserted inside the through-hole 491 of the cylindrical body 490), the inner diameter d2 of the through-hole 425 is larger than the inner diameter d1a of the through-hole 491 at the center portion 490a. Therefore, the area S2 of the through-hole 425 in the transverse cross-section of the first small diameter portion 423 of the stopper 420 on the distal end side is larger than the area S1a of the through-hole 491 in the transverse cross-section of the center portion 490a of the cylindrical body 490. Similarly, in the first small diameter portion 423 of the stopper 420 on the proximal end side (the part inserted inside the through-hole 491 of the cylindrical body 490), the inner diameter d3 of the through-hole 425 is larger than the inner diameter d1a of the through-hole 491 at the center portion 490a of the cylindrical body 490. Therefore, the area S3 of the through-hole 425 in the transverse cross-section of the first small diameter portion 423 of the stopper 420 on the proximal end side is larger than the area S1a of the through-hole 491 in the transverse cross-section of the center portion 490a of the cylindrical body 490.

In the present embodiment, the outer diameter of the center portion 490a is larger than the diameter of the distal end portion 490b, and larger than the outer diameter of the proximal end portion 490c. Therefore, in the cylindrical body 490 accommodated in the substantially hollow cylinder-shaped cylindrical body accommodating space 215 of the housing 210, the outer peripheral surface of the center portion 490a makes contact with, or is close to, the inner peripheral surface of the cylindrical body accommodating space 215, while the outer peripheral surfaces of the distal end portion 490b and the proximal end portion 490c are separated from the inner peripheral surface of the cylindrical body accommodating space 215.

The fixing mechanism 200B of the third embodiment includes a pressing member 440 instead of the pressing member 240 of the first embodiment. The pressing member 440 has a shape in which the part on the upper side of the through-hole 241 of the pressing member 240 of the first embodiment has been removed. That is, the pressing member 440 is disposed below the center portion 490a of the cylindrical body 490, and in the released state described above, the upper surface of the pressing member 440 makes contact with, or is close to, the outer peripheral surface of the center portion 490a of the cylindrical body 490.

Like the first embodiment, the pressing member 440 is capable of sliding along the up-down direction (Y-axis direction), which is a direction orthogonal to the axial direction (Z-axis direction) of the cylindrical body 490. The pressing member 440, which is capable of sliding in the up-down direction, can be positioned in the non-pressing position P5 shown in FIG. 25, which substantially does not press the cylindrical body 490, and the pressing position P6 shown in FIG. 26, which is upwardly displaced form the non-pressing position P5. Here, the state of substantially not pressing includes, in addition to a state where the upper surface of the pressing member 440 is separated from the outer peripheral surface of the cylindrical body 490, a state where the upper surface of the pressing member 440 is in close contact with the outer peripheral surface of the cylindrical body 490.

On the other hand, when the pressing member 440 is positioned in the pressing position P6 (FIG. 26), the pressing member 440 upwardly presses the center portion 490a of the cylindrical body 490 from the outer peripheral side and elastically deforms the center portion 490a. In this state, a contact position CP1, being a lower side part of the inner peripheral surface of the center portion 490a, and a contact position CP3, being an upper side part of the inner peripheral surface of the center portion 490a, which are on the inner peripheral surface of the through-hole 491 of the elastically deformed center portion 490a, are pressed against the long medical device, and the sliding of the long medical device in the front-rear direction is regulated as a result of the long medical device being fixed by the fixing mechanism 200B by being held between the two contact positions CP1 and CP3.

Here, as mentioned above, the area S2 of the through-hole 425 in the transverse cross-section of the first small diameter portion 423 of the stopper 420 on the distal end side and the area S3 of the through-hole 425 in the transverse cross-section of the first small diameter portion 423 of the stopper 420 on the proximal end side are larger than the area S1a of the through-hole 491 in the transverse cross-section of the center portion 490a of the cylindrical body 490. Therefore, even in a state in which the long medical device is fixed as a result of the pressing member 440 pressing the center portion 490a of the cylindrical body 490, the pressing of the long medical device against the inner peripheral surfaces of the stoppers 420 is suppressed, and damage to the long medical device can be more effectively suppressed. Note that such an effect can be obtained in a solid long medical device such as a guide wire, but can be especially obtained in a long medical device having a hollow portion such as a catheter.

### C. Modifications:

The technique disclosed herein is not limited to the embodiment described above, and various modifications can be made within a scope that does not depart from the gist thereof. For example, the following modifications can be made.

The configurations of the medical connector 10, the opening/closing mechanisms 100 and 300, and the fixing mechanism 200 in the embodiments described above are merely examples, and various modifications are possible. For example, in the first embodiment described above, the protrusion 139 that is inserted into the hollow portion of the biasing member 106 is formed on the surface of the through-member 130 facing the biasing member 106, but the protrusion 139 does not have to be formed. The protrusion 232 of the force transmission member 230 can be similarly omitted.

In the first embodiment described above, although the biasing member 106 is disposed in a position facing the operating member 140 in the front-rear direction, the biasing member 106 may be disposed in another position. The arrangement of the biasing member 306 of the second embodiment described above may be similarly changed.

In the second embodiment described above, although the operating member 340 includes two pieces (the first piece 340U and the second piece 340L), the operating member 340 may be configured by one piece.

In the embodiments described above, although a double knock mechanism is used as the holding mechanism 260 that switches between the state in which the force transmission member 230 is held in the retracted position P7 and the state in which the force transmission member 230 is held in the advanced position P8, another mechanism such as a heart cam mechanism may also be used as the holding mechanism 260.

In the embodiments described above, the housing 210 and the stoppers 220 and 240 may be an integrated member.

The dimensions and materials of each member in the embodiments above are merely examples, and various modifications are possible.

In the embodiments described above, although the medical connector 10 includes both the opening/closing mechanism 100 (or the opening/closing mechanism 300) and the fixing mechanism 200, the medical connector 10 may include only one of the opening/closing mechanism 100 (or the opening/closing mechanism 300) and the fixing mechanism 200.

### DESCRIPTION OF REFERENCE NUMERALS

10: Medical connector (first embodiment)
11: Main pipe portion
12: Branched pipe portion
13: Lumen
14: Lumen
20: Rotator
100: Opening/closing mechanism
106: Biasing member
110: Housing
111: Proximal end side opening
112: Distal end side opening
113: Lumen
114: Partition wall
114A: Through-hole
115: Guide groove
116: Operating member accommodating space
117: Groove
118: Biasing member accommodating space
119: Protrusion
120: Hemostasis valve
121: Slit
122: Through-hole
128: Boss
130: Through-member
131: Main body portion
132: Through-hole
133: Flange portion
134: Guide protrusion
135: Guide protrusion
136: Upper wall portion
137: Motion restricting groove
137A: Closed position portion
137B: First top portion
137C: Open holding portion
137D: Second top portion
139: Protrusion
140: Operating member
141: Operation accepting portion
142: Connection portion
143: Distal end surface
144: Guide groove
145: Recess
172: Pin
173: End portion
200: Fixing mechanism
210: Housing
211: Proximal end side opening
212: Distal end side opening
213: Lumen
214: Retraction space
215: Cylindrical body accommodating space
216: Member accommodating space
220: Stopper
221: Main body portion
222: Second small diameter portion
223: First small diameter portion
224: Recess
230: Force transmission member
232: Protrusion
233: Surface
234: Upper surface
235: Guide groove
240: Pressing member
241: Through-hole
244: Lower surface
245: Guide protrusion
250: Outer cylinder
253: Outer cylinder end surface cam
254: Shallow groove portion
255: Deep groove portion
260: Holding mechanism
262: Biasing member
270: Rotor
273: Protrusion
274: Recess
280: Operating member
281: Sliding contactor
282: Operating member end surface cam
290: Cylindrical body
291: Through-hole
10A: Medical connector (second embodiment)
300: Opening/closing mechanism
306: Biasing member
310: Housing
311: Proximal end side opening
312: Distal end side opening
313: Lumen
314: Partition wall
314A: Through-hole
316: Through-hole for operating member
317: Groove
318: Distal end side member
319: Proximal end side member
320: Hemostasis valve
321: Slit
322: Through-hole
328: Boss
330: Through-member
331: Main body portion
332: Through-hole
333: Connection portion
334: Recess
335: Through-member side contact surface
337: Motion restricting groove
337A: Closed position portion
337B: First top portion
337C: Open holding portion
337D: Second top portion
340: Operating member
340L: Second piece
340U: First piece
341: Connection part
342: Arm portion
345: Operating member side contact surface
372: Pin
373: End portion
10B: Medical connector (third embodiment)
200B: Fixing mechanism
420: Stopper
421: Main body portion
422: Second small diameter portion
423: First small diameter portion
424: Recess
425: Through-hole
440: Pressing member
490: Cylindrical body
490a: Center portion
490b: Distal end portion
490c: Proximal end portion
491: Through-hole
CP 1: Contact position
CP2: Contact position
CP3: Contact position
GC: Guiding catheter
GW: Guide wire

## Claims

1. An opening/closing mechanism for a hemostasis valve comprising:
a tubular housing formed having a lumen that communicates with a distal end side opening and a proximal end side opening;
a hemostasis valve attached inside the housing and normally in a closed state, which is switched to an open state when pressed from a proximal end side such that a through-hole is formed that communicates with the distal end side opening of the housing;
a through-member that is accommodated inside the housing so as to be capable of sliding along a first direction, being an extending direction of the lumen further toward a proximal end side than the hemostasis valve, and is formed having a through-hole that communicates with the proximal end side opening of the housing, the through-member being capable of being positioned in a first position that places the hemostasis valve in the closed state, and a second position, being a position further toward a distal end side along the first direction than the first position, which places the hemostasis valve in the open state by pressing the hemostasis valve, and communicates the through-hole of the hemostasis valve and the through-hole of the through-member;
an operating member that is accommodated inside the housing so as to be capable of sliding along a second direction that is not parallel to the first direction, in a state where one part is exposed from the housing, and is capable of pressing and displacing the through-member toward a distal end side by sliding along the second direction toward an inner side of the housing; and
a biasing member that biases the through-member toward a proximal end side; wherein
a surface of the through-member is formed having a motion restricting groove that is connected over an entire circumference,
the opening/closing mechanism for a hemostasis valve is further provided with a pin that is attached to the housing such that an end portion of the pin is loosely fitted to the motion restricting groove, and
the motion restricting groove is formed having a closed position portion, to which the end portion of the pin is fitted without restricting a movement of the through-member toward a distal end side when the through-member is in a first state at the first position, a first top portion to which the end portion of the pin is fitted without restricting a movement of the through-member toward a proximal end side when the through-member is in a second state, in which the through-member has been pressed from the first state by the operating member and reaches a position further toward a distal end side than the second position, an open holding portion to which the end portion of the pin is fitted such that a movement of the through-member toward a proximal end side is restricted when the through-member is in a third state, in which the through-member has been displaced from the second state by a biasing force of the biasing member to the second position, and a second top portion to which the end portion of the pin is fitted without restricting a movement of the through-member toward a proximal end side when the through-member is in a fourth state, in which the through-member has been pressed from the third state by the operating member and reaches a position further toward a distal end side than the second position.

2. The opening/closing mechanism for a hemostasis valve according to claim 1, wherein
the biasing member has a cylindrical shape, and
a surface of the through-member facing the biasing member is formed having a protrusion that is inserted into a hollow portion of the biasing member.

3. The opening/closing mechanism for a hemostasis valve according to claim 1 or 2, wherein
an angle formed between the first direction and the second direction is 35 degrees or more and 55 degrees or less.

4. The opening/closing mechanism for a hemostasis valve according to claim 3, wherein
the biasing member and the operating member are disposed so as to face each other in the first direction.

5. The opening/closing mechanism for a hemostasis valve according to claim 3 or 4, wherein
the through-member comprises a main body portion that is formed having the through-hole, and a flange portion that protrudes from the main body portion in a third direction, which is orthogonal to the first direction, and
the operating member makes contact with a surface of the flange portion on a proximal end side, while also being capable of a relative sliding movement with respect to the through-member along the third direction.

6. The opening/closing mechanism for a hemostasis valve according to claim 1 or 2, wherein
an angle formed between the first direction and the second direction is substantially 90 degrees.

7. The opening/closing mechanism for a hemostasis valve according to claim 6, wherein
the operating member is formed of a first piece and a second piece that face each other in the second direction, and
the first piece and the second piece slide along the second direction so as to approach each other, which causes the through-member to be pressed and displaced toward a distal end side.

8. The opening/closing mechanism for a hemostasis valve according to claim 7, wherein
the first piece and the second piece each have an operating member side contact surface, being a surface that is parallel to a fourth direction which is orthogonal to both the first direction and the second direction, that is not parallel to both the first direction and the second direction, and
the through-member has a through-member side first contact surface, being a surface that is parallel to the fourth direction and not parallel to both the first direction and the second direction, that makes contact with the operating member side contact surface of the first piece, and a through-member side second contact surface, being a surface that is parallel to the fourth direction and not parallel to both the first direction and the second direction, that makes contact with the operating member side contact surface of the second piece.

9. A fixing mechanism for a long medical device, comprising:
a tubular housing formed having a lumen that communicates with a distal end side opening and a proximal end side opening;
a flexible cylindrical body attached inside the housing and formed having a through-hole into which the long medical device is inserted, the through-hole communicating with the distal end side opening and the proximal end side opening of the housing;
a pressing member that is accommodated inside the housing and capable of sliding along a sixth direction, which is a direction orthogonal to a fifth direction that is parallel to an axis of the cylindrical body, the pressing member being capable of being positioned in a fifth position, and a sixth position that is displaced from the fifth position along the sixth direction, at which a part of the cylindrical body excluding both end portions is deformed by being pressed from an outer peripheral side;
an operating member that is capable of sliding along a seventh direction that is not parallel to the fifth direction;
a force transmission member that is disposed so as to be capable of sliding in the seventh direction between the pressing member and the operating member, and that transmits, to the pressing member, a force that moves the operating member along the seventh direction so as to approach the pressing member, the force transmission member being capable of being positioned in a seventh position that positions the pressing member in the fifth position, and an eighth position that presses the pressing member and positions the pressing member in the sixth position; and
a holding mechanism that switches states between a state in which the force transmission member is held in the seventh position and a state in which the force transmission member is held in the eighth position each time the operating member slides along the seventh direction; wherein
when the pressing member is positioned in the sixth position, an inner peripheral surface of the deformed cylindrical body is pressed against the long medical device, which causes the long medical device to become fixed by the cylindrical body.

10. The fixing mechanism for a long medical device according to claim 9, wherein
the holding mechanism comprises a cylindrical biasing member that biases the force transmission member in a direction approaching the pressing member, and
a surface of the force transmission member facing the biasing member is formed having a protrusion that is inserted into a hollow portion of the biasing member.

11. The fixing mechanism for a long medical device according to claim 9 or 10, wherein
an angle formed between the fifth direction and the seventh direction is 35 degrees or more and 55 degrees or less.

12. The fixing mechanism for a long medical device according to claim 11, wherein
the force transmission member makes contact with a surface of the pressing member, while also being capable of a relative sliding movement with respect to the pressing member along the fifth direction.

13. A fixing mechanism for a long medical device, comprising:
a tubular housing formed having a lumen that communicates with a distal end side opening and a proximal end side opening, and which is capable of accommodating the long medical device in the lumen;
a flexible cylindrical body attached inside the housing and formed having a first through-hole into which the long medical device is inserted, the first through-hole communicating with the distal end side opening and the proximal end side opening of the housing;
a distal end side connection portion that connects a distal end portion of the cylindrical body to the housing, and is formed having a second through-hole that communicates with the first through-hole of the cylindrical body;
a proximal end side connection portion that connects a proximal end portion of the cylindrical body to the housing, and is formed having a third through-hole that communicates with the first through-hole of the cylindrical body; and
a pressing member that presses a pressed part of the cylindrical body excluding both end portions from a direction that is not parallel to an axis of the cylindrical body, and which is accommodated inside the housing; wherein
an area of the second through-hole in a transverse cross-section of the fixing mechanism of the distal end side connection portion and an area of the third through-hole in the transverse cross-section of the proximal end side connection portion are larger than an area of the first through-hole in the transverse cross-section of the pressed part.

14. The fixing mechanism for a long medical device according to claim 13, wherein
the pressing member is accommodated inside the housing and capable of sliding along a sixth direction, which is a direction orthogonal to a fifth direction that is parallel to an axis of the cylindrical body, and is capable of being positioned in a fifth position, and a sixth position that is displaced from the fifth position along the sixth direction, at which the pressed part of the cylindrical body is deformed by being pressed from an outer peripheral side, and
the fixing mechanism further comprises
an operating member that is capable of sliding along a seventh direction that is not parallel to the fifth direction,
a force transmission member that is disposed so as to be capable of sliding in the seventh direction between the pressing member and the operating member, and that transmits, to the pressing member, a force that moves the operating member along the seventh direction so as to approach the pressing member, the force transmission member being capable of being positioned in a seventh position that positions the pressing member in the fifth position, and an eighth position that presses the pressing member and positions the pressing member in the sixth position, and
a holding mechanism that switches states between a state in which the force transmission member is held in the seventh position and a state in which the force transmission member is held in the eighth position each time the operating member slides along the seventh direction, and
when the pressing member is positioned in the sixth position, an inner peripheral surface of the deformed cylindrical body is pressed against the long medical device, which causes the long medical device to become fixed by the cylindrical body.

15. The fixing mechanism for a long medical device according to claim 14, wherein
the holding mechanism comprises a cylindrical biasing member that biases the force transmission member in a direction approaching the pressing member, and
a surface of the force transmission member facing the biasing member is formed having a protrusion that is inserted into a hollow portion of the biasing member.

16. The fixing mechanism for a long medical device according to claim 14 or 15, wherein
an angle formed between the fifth direction and the seventh direction is 35 degrees or more and 55 degrees or less.

17. The fixing mechanism for a long medical device according to claim 16, wherein
the force transmission member makes contact with a surface of the pressing member, while also being capable of a relative sliding movement with respect to the pressing member along the fifth direction.

18. A medical connector comprising:
the opening/closing mechanism for a hemostasis valve according to any one of claims 1 to 8; and
the fixing mechanism for a long medical device according to any one of claims 9 to 17.
